# EUROPEAN PATENT APPLICATION

(11) **EP 4 194 089 A2**
(43) Date of publication of application: **14.06.2023**
(21) Application number: 22178854.0
(22) Date of filing: 14.06.2022
(51) Int. Cl.: B01J 31/02, B01J 31/18, B01J 31/22, B01J 37/34, C07D 209/58, C07D 219/14, C07D 265/38

(54) **CATALYST SYSTEM FOR SUZUKI CROSS-COUPLING REACTIONS COMPRISING A PHOTOREDOX COMPONENT**

(30) Priority: 19.07.2021 US 202163203349 P
(71) Applicant: Heraeus Deutschland GmbH & Co. KG, 63450 Hanau (DE); New Iridium, Inc., Boulder, CO 80303-1961 (US)
(72) Inventor: Walter, Philipp, 63450 Hanau (DE); Kolvenbach, Robin, 6850 MENDRISIO (CH); Gock, Michael, 63450 Hanau (DE); Elder, William Zachary, FORT COLLINS, CO 80526 (US); Federico, Celia, BOULDER, CO 80303-1961 (US); Boston, David J., BOULDER, CO 80303-1961 (US); Vora, Harit, BOULDER, CO 80303-1961 (US); Cutcliffe, Brent, BOULDER, CO 80303-1961 (US); Lim, Chern-Hooi, BOULDER, CO 80303-1961 (US)
(74) Representative: Heraeus IP

(57) **Abstract**

The present invention relates to a composition, comprising
- a palladium compound which is a palladium salt or a palladium complex or a mixture thereof, and
- a polycyclic compound of Formula (I), (II) or (III):

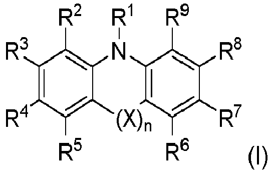

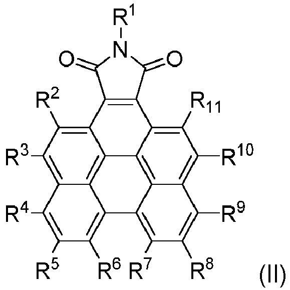

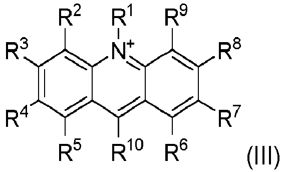

## Description

### FIELD OF THE INVENTION

The invention relates to a catalyst system for light-assisted Suzuki cross-coupling reactions.

### BACKGROUND OF THE INVENTION

Cross-coupling reactions are a frequently used and important synthetic tool in organic chemistry, both for joining two fragments via a carbon-carbon bond (carbon-carbon cross-coupling reactions) and a carbon-heteroatom bond (carbon-heteroatom cross-coupling reactions).

Different types of cross-coupling reactions such as Suzuki cross-coupling (also known as Suzuki-Miyaura cross-coupling), Heck cross-coupling, Stille cross-coupling and Negishi cross-coupling reactions are known. Typically, these cross-coupling reactions are catalyzed by palladium-containing catalysts. In 2010, the Nobel Prize in Chemistry was awarded jointly to R.F. Heck, E. Negishi and A. Suzuki for palladium-catalyzed cross-couplings in organic synthesis.

Cross-coupling reactions are frequently used in drug synthesis. Reviews on this topic are provided by H.C. Shen, "Selected Applications of Transition Metal-Catalyzed Carbon-Carbon Cross-Coupling Reactions in the Pharmaceutical Industry', pp. 25-96, in "Applications of Transition Metal Catalysis in Drug Discovery and Development: An Industrial Perspective", Ed.: M.L. Crawley and B.M. Trost, 2012, John Wiley & Sons; and Q. Gu et al., "Palladium catalyzed C-C and C-N bond forming reactions: An update on the synthesis of pharmaceuticals from 2015-2020", Org. Chem. Front., 2021, 8, pp. 384-414.

In a Suzuki cross-coupling reaction, an organoboron species is typically reacted with an organohalide or organopseudohalide in the presence of a palladium compound acting as a catalyst. A review on palladium-catalyzed Suzuki cross-coupling reactions of organoboron compounds is provided by A. Suzuki and N. Miyaura, Chem. Rev., 1995, pp. 2457-2483. A variety of different organoboron reagents for use in Suzuki cross-coupling reactions have meanwhile been developed and are commercially available. See e.g. A.J. Lennox and G.C. Lloyd-Jones, "Selection of boron reagents for Suzuki-Miyaura coupling", Chem. Soc. Rev., 2014, pp. 412-443.

Palladium in the oxidation state 0 (Pd(0)) is considered to be the catalytically active species in Suzuki cross-coupling reactions, e.g. in the form of L-Pd(0) or L₂Pd(0), wherein L is a ligand which binds to the Pd atom.

Typically, the most active cross-coupling catalysts are Pd complexes with sterically demanding, electron-rich phosphine or N-heterocyclic carbene (NHC) ligands. The catalytically active species can be generated *in situ* in the reaction medium by bringing into contact a palladium salt (such as Pd(OAc)₂) and appropriate phosphine or N-heterocyclic carbene compounds. Such phosphine or N-heterocyclic carbene compounds are known to the skilled person and commercially available. Meanwhile, kits containing a number of different phosphine or N-heterocyclic carbene compounds are offered by commercial suppliers for use in palladium-catalyzed cross-coupling reactions (such as Suzuki cross-coupling).

However, the costs of these specialized ligands are often quite high which is why the addition of excess ligand to a Pd precursor may adversely affect economic process efficiency. Furthermore, in many cross-coupling reactions, the optimal Pd to ligand ratio is 1:1, with the active species proposed to be monoligated Pd(0). Therefore, well-defined Pd(II) precatalysts containing one molar equivalent of ancillary ligand to facilitate cross-coupling reactions are meanwhile frequently used. Reviews on palladium compounds acting as precatalysts for cross-coupling reactions are provided by N. Hazari et al., "Well-defined nickel and palladium precatalysts for cross-coupling", Nature Reviews Chemistry, 1,0025 (2017), https://doi.org/10.1038/s41570-017-0025, and K.H. Shaughnessy, Israel Journal of Chemistry, Vol. 60, Issue 3-4, pp. 180-194. Exemplary Pd precatalysts include palladacycle precatalysts (also known as Buchwald precatalysts) and PEPPSI^{®} precatalysts ("Pyridine-Enhanced Precatalyst Preparation Stabilization and Initiation"). Meanwhile, a wide variety of palladium precatalysts for use in cross-coupling reactions is commercially available, e.g. Buchwald palladacycle precatalyst kits from Strem Chemicals and Sigma-Aldrich. Further information about commercially available palladium precatalysts for cross-coupling reactions is provided by the Sigma-Aldrich brochure *"Application Guide for Palladium Catalyzed Cross-Coupling Reactions"* (available at https://www.sigmaaldrich.com/content/dam/sigma-aldrich/docs/Aldrich/Application_Notes/1/application-guide-for-palladium-catalyzed-cross-coupling.pdf) and the Strem Chemicals brochure *"Buchwald Ligands and Precatalysts"* (available at https://www.strem.com/uploads/resources/documents/buchwaldligprecat.pdf).

In Suzuki cross-coupling reactions, reactivity of the aryl halide reactant decreases in the order of I > Br >> Cl. For cost efficiency reasons, aryl chloride reactants would typically be preferred over aryl bromide and aryl iodide reactants. Furthermore, some heteroaryl halide reactants such as pyridyl chloride show low reactivity in Suzuki cross-coupling reactions. Unless no improved catalytic system is identified, low activity of the reactants is typically overcome by increasing reaction temperature which, however, may amplify side reactions and decrease product yield of the Pd-catalyzed Suzuki cross-coupling reaction.

Accordingly, there is still a need for improved catalyst systems which efficiently catalyze Suzuki cross-coupling reactions even if aryl chlorides or heteroaryl chlorides (such as pyridyl chlorides) are used as reactants.

Recently, light-induced photoredox catalysis has become an important tool for the design and development of a variety of radical reactions under mild reaction conditions. A review on photoredox catalysis in organic chemistry is provided by M.H. Shaw et al., J. Org. Chem., 2016, 81, pp. 6898-6926.

US 10,975,171 B2 discloses phenoxazine and dihydrophenazine photoredox catalysts which might be used in atom transfer radical addition/polymerization, dehalogenation, cycloaddition, cyclization, dimerization, coupling, reduction, ring-opening, alkylation, arylation, oxygenation and radical addition. As indicated in US 10,975,171 B2, the reaction medium is preferably free of a metal or metalloid.

Dimethyl dihydroacridines as photoredox catalysts in atom transfer radical polymerization of acrylate monomers is described by C. Lim et al., Angew. Chem. Int. Ed. Engl., 2020, 59(8), pp. 3209-3217.

C. Lim et al., Chemistry, 2017, 23(46), pp. 10962-10968, describe strongly reducing visible light organic photoredox catalysts (such as N-aryl phenoxazines and dihydrophenazines) as an alternative to iridium and ruthenium photoredox catalysts.

C. Lim et al, J. Am. Chem. Soc., 2020, 142, 31, pp. 13573-13581, describe a light-induced Birch reduction in the presence of a benzoperylene monoimide acting as a photoredox catalyst.

A combination of transition metal catalysts and photoredox catalysts is described by J. Twilton et al., "The merger of transition metal and photocatalysis", Nat Rev Chem, 1, 0052 (2017), https://doi.org/10.1038/s41570-017-0052.

A review on cooperative photoredox and palladium catalysis is provided by N.D.P. Singh et al., Catal. Sci. Technol., 2021, 11, pp. 742-767.

K. Mori et al., Chem. Comm., 2014, 50, pp. 14501-14503, describe a light-enhanced Suzuki cross-coupling reaction by cooperative photocatalysis with a Ru-Pd bimetallic complex. The Ru bipyridine moiety represents the photocatalytically active part, while the Pd bipyrimidine moiety represents the precatalyst part which is transformed into the active Pd(0) species. So, the photocatalytically active component and the palladium precatalyst component are present within the same molecule. However, synthesis of compounds which contain both the photocatalytically active component and the palladium precatalyst component within the same molecule might be challenging. Furthermore, as indicated in the publication of K. Mori et al., no enhancement of catalytic activity has been observed when using a physical mixture of the Pd-free Ru complex and the Ru-free Pd complex (i.e. a mixture of [(bpy)₂Ru(bpm)](PF₆)₂ and (bpy)PdCl₂). This suggests that the electron transfer from Ru to Pd moiety is crucial for attaining enhanced photocatalytic activity and requires the photocatalytically active component and the palladium precatalyst component to be present within the same molecule.

X. Li et al., Scientific Reports, 3:1743, DOI: 10.1038/srep01743 describe a room-temperature Suzuki carbon-carbon cross-coupling reaction over heterogeneous Pd catalysts by light-mediated catalyst activation. A stimulated electron transfer at the metal-semiconductor interface from optically active mesoporous carbon nitride nanorods to Pd nanoparticles is employed.

As mentioned above, (hetero)aryl chlorides may show low reactivity in Suzuki cross-coupling reactions. Unless no improved catalytic system is identified, low activity of the reactants is typically overcome by increasing reaction temperature which, however, may amplify side reactions and decrease product yield of the Pd-catalyzed Suzuki cross-coupling reaction.

Accordingly, an object of the present invention is to provide a catalyst system which efficiently catalyzes Suzuki cross-coupling reactions, in particular if (hetero)aryl chlorides are used as a reactant and/or the reaction is carried out at relatively low temperature.

### SUMMARY OF THE INVENTION

The invention provides a composition, comprising
(i) a palladium compound which is selected from a palladium salt or a palladium complex or a mixture thereof,
(ii) a polycyclic compound of Formula (I), (II) or (III): 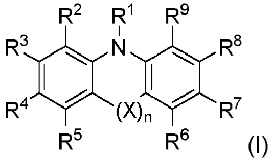 wherein in Formula (I)
   n is 0 or 1,
   X is O, C(R¹⁰)(R¹¹), or N(R¹²),
   R¹ to R¹² are selected independently from each other from hydrogen, an aryl group and a C₁₋₁₀alkyl group, the aryl group and/or the C₁₋₁₀alkyl group optionally being substituted with one or more substituents R¹³,
   wherein the one or more substituents R¹³, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 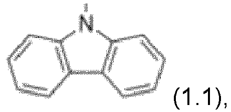 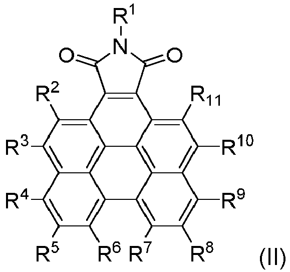
   wherein in Formula (II)
   R¹ is selected from a C₁₋₁₆alkyl group and an aryl group, the C₁₋₁₆alkyl group and/or the aryl group optionally being substituted with one or more substituents R¹², and/or the C₁₋₁₆alkyl group optionally being interrupted by one or more oxygen atoms;
   R² to R¹¹ are selected independently from each other from hydrogen, a C₁₋₁₆alkyl group, an aryl group, a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms; or
   at least two of the residues R² to R¹¹ which are adjacent to each other form together with the carbon atoms to which they are attached a five- or six-membered aromatic ring while the remaining residues (i.e. those of the residues R² to R¹¹ which do not form a ring) are selected independently from each other from hydrogen, a C₁₋₁₆alkyl group, an aryl group and a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms;
   wherein the one or more substituents R¹², if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 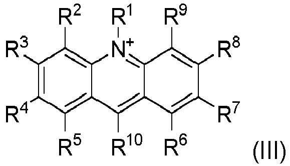 wherein in Formula (III)
   R¹ is selected from a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
   R² to R⁹ are selected independently from each other from hydrogen, a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
   R¹⁰ is an aryl group which is optionally substituted with one or more substituents R¹², wherein the one or more substituents R¹¹, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl),
   wherein the one or more substituents R¹², if present, are selected independently from each other from a C₁₋₆alkyl group and an aryl group (e.g. a phenyl group), wherein the aryl group is optionally substituted with one or more C₁₋₆alkyl groups.

In the present invention, it has been realized that the composition comprising a palladium compound and a polycyclic compound of Formula (I), (II) or (III) represents an efficient catalyst system for light-assisted Suzuki cross-coupling reactions.

Accordingly, the present invention also provides a method of performing a Suzuki cross-coupling reaction, wherein a first reactant is reacted with a second reactant under irradiation of light in the presence of the composition according to the present invention, the first reactant comprising an aromatic or N-heteroaromatic ring to which a boronic acid or boronic acid ester group is attached, and the second reactant comprising an aromatic or N-heteroaromatic ring to which a halogen (CI, Br, I), a pseudohalogen (cyano, cyanate, isocyanate), or a sulfonate is attached. Examples of sulfonates comprise hydrocarbyl sulfonate like, for example, aryl sulfonate and alkyl sulfonate. Such sulfonate may or may not be substituted with halogen, e.g. trifluoromethane sulfonate.

As discussed below in further detail, *"under irradiation of light"* refers to light provided by an external light source (e.g. lamps such as UV or UV/VIS lamps, LEDs, laser, etc.).

The present invention also relates to the use of the composition outlined above and described below in further detail as a catalyst system for a light-assisted Suzuki carbon-carbon cross-coupling reaction. As known to the skilled person, *"light assisted"* means that the reaction medium in which the Suzuki carbon-carbon cross-coupling reaction is carried out is irradiated by an external light source (e.g. lamps such as UV or UV/VIS lamps, LEDs, laser, etc.).

### DETAILED DESCRIPTION

As outlined above, the composition of the present invention may comprise a polycyclic compound of Formula (I).

If one or more of the residues R¹ to R¹² in Formula (I) are an aryl group, it can be a monoaryl group (e.g. a phenyl or naphthyl group) or a diaryl group (e.g. a biphenyl group).

Preferably, R¹ in Formula (I) is an aryl group, which can be a monoaryl group (e.g. a phenyl or naphthyl group) or a diaryl group (e.g. a biphenyl group).

Preferably, R¹ in Formula (I) is selected from a phenyl group, a naphthyl group and a biphenyl group, each of which is optionally substituted with one or more substituents R¹³, wherein R¹³ has the same meaning as outlined above. Accordingly, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and N-carbazolyl of Formula (1.1)

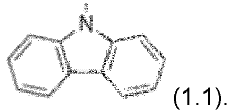

If in Formula (I) n is 1 and X is O, the polycyclic compound has the following Formula (Ia): 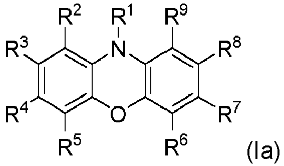 wherein R¹ to R⁹ have the same meanings as outlined above in Formula (I).

In a preferred embodiment of Formula (Ia),
R¹ is a naphthyl group (preferably a 1-naphthyl group) which is optionally substituted with one or more substituents R¹³,
R², R³, R⁵, R⁶, R⁸ and R⁹ are selected independently from each other from hydrogen and a
C₁₋₆alkyl group,
R⁴ and R⁷ are selected independently from each other from a phenyl group, a biphenyl group and a naphthyl group, each of which is optionally substituted with one or more substituents R¹³, wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

Exemplary compounds of Formula (Ia) are those of the following Formulas (I-1), (I-2), (I-3) and (I-4):

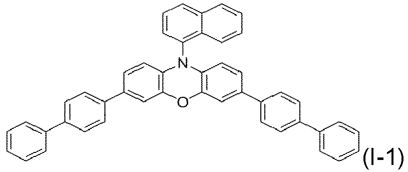

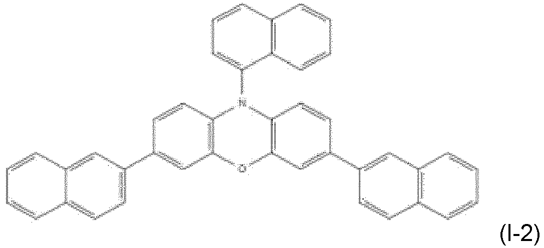

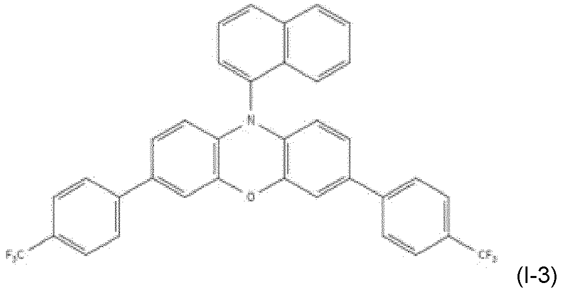

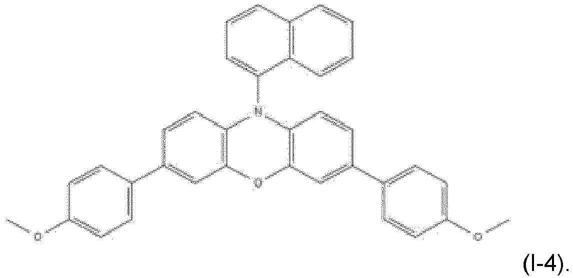

If in Formula (I) n is 1 and X is C(R¹⁰)(R¹¹), the polycyclic compound has the following Formula (Ib): 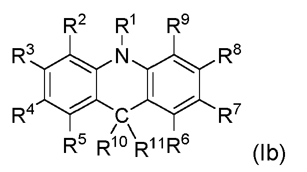 wherein R¹, R² to R⁹ and R¹⁰ to R¹¹ have the same meanings as outlined above in Formula (I).

In a preferred embodiment of Formula (Ib),
R¹⁰ and R¹¹ are selected independently from each other from hydrogen and a C₁₋₆alkyl group,
R¹ is selected from a naphthyl group (preferably a 1-naphthyl or 2-naphthyl group), a phenyl group and a biphenyl group, each of which is optionally substituted with one or more substituents R¹³,
R², R³, R⁵, R⁶, R⁸ and R⁹ are selected independently from each other from hydrogen and a C₁₋₆alkyl group,
R⁴ and R⁷ are selected independently from each other from a phenyl group, a naphthyl group and a biphenyl group, each of which is optionally substituted with one or more substituents R¹³, wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).

Exemplary compounds of Formula (Ib) have the following Formulas (I-5.1) to (I-5.7):

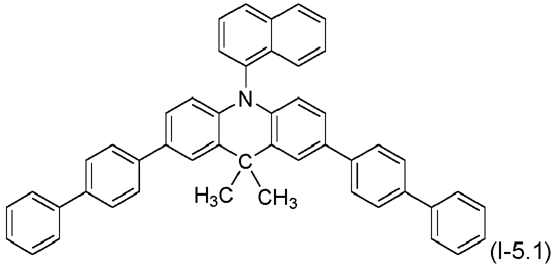

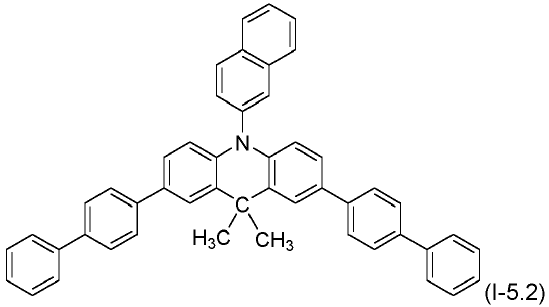

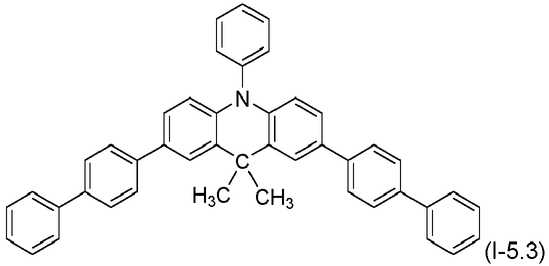

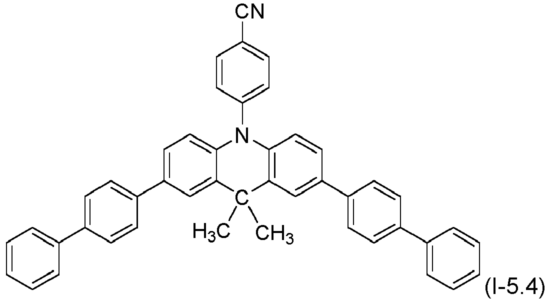

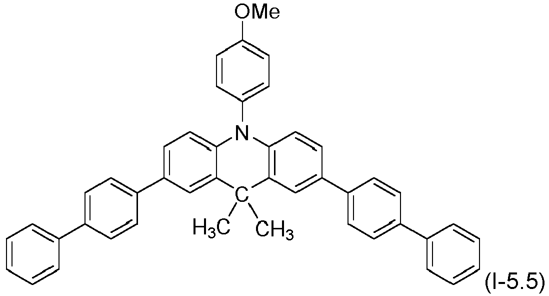

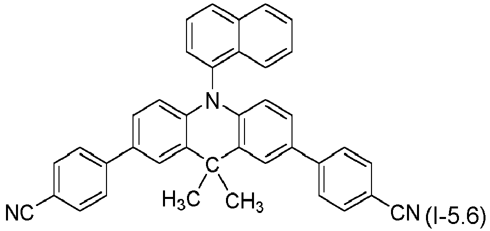

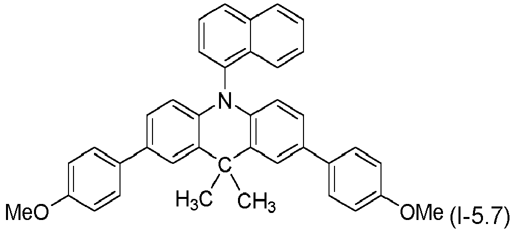

If in Formula (I) n is 1 and X is N(R¹²), the polycyclic compound has the following Formula (Ic): 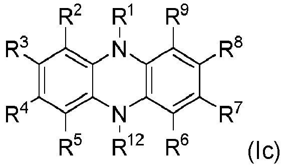 wherein R¹, R² to R⁹ and R¹² have the same meanings as outlined above in Formula (I).

In a preferred embodiment of Formula (Ic),
R¹ and R¹² are selected independently from each other from a phenyl group, a naphthyl group (such as a 2-naphthyl group or a 1 naphthyl group) and a biphenyl group, each of which is optionally substituted with one or more substituents R¹³,
R² to R⁹ are selected independently from each other from hydrogen and a C₁₋₆alkyl group, or at least two, preferably all of the residues R³, R⁴, R⁷, and R⁸ are a phenyl group which is optionally substituted with one or more substituents R¹³ and the residues R², R⁵, R⁶ and R⁹ are selected independently from each other from hydrogen and a C₁₋₆alkyl group,
wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

Exemplary compounds of Formula (Ic) are those of the following Formulas (1-6) and (I-7):

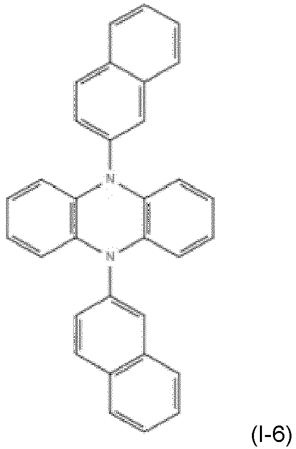

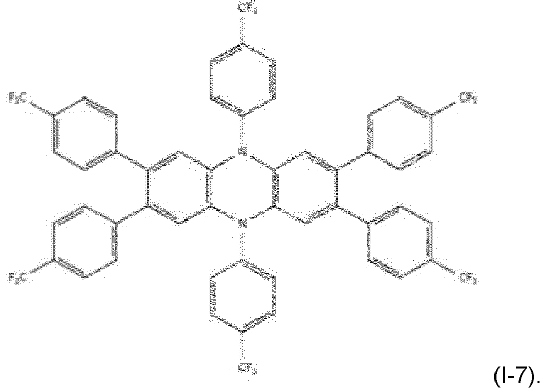

If in Formula (I) n is 0, the polycyclic compound has the following Formula (Id): 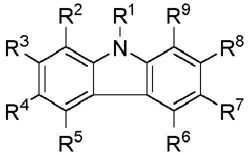 wherein R¹ and R² to R⁹ have the same meanings as outlined above in Formula (I).

In a preferred embodiment of Formula (Id),
R¹ is selected from a naphthyl (e.g. 1-naphthyl or 2-naphthyl) group, a phenyl group and a biphenyl group, each of which is optionally substituted with one or more R¹³,
R² to R⁹ are selected independently from each other from hydrogen, a C₁₋₆alkyl group, a phenyl group, biphenyl group and a naphthyl group, the phenyl group, the biphenyl group and/or the naphthyl group optionally being substituted with one or more substituents R¹³,
wherein, if present, the one or more substituents R¹³, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1)

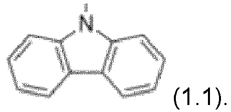

An exemplary compound of Formula (Id) has the following Formula (I-8):

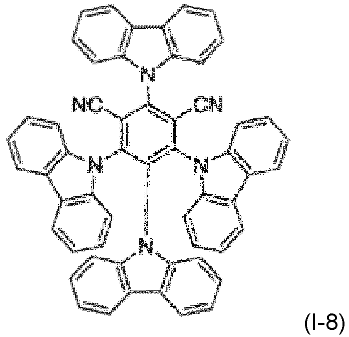

As outlined above, the composition of the present invention may comprise a polycyclic compound of Formula (II).

As mentioned above, one or more of the residues R² to R¹¹ in Formula (II) can be a heteroaryl group. If present, the heteroaryl group preferably comprises or is a five- or six-membered aromatic ring containing one or more (preferably up to three) heteroatoms being selected from oxygen, nitrogen and sulfur. Exemplary heteroaryl groups are pyridinyl, pyridazinyl, pyrimidinyl, pyrazinyl, oxazolyl, thiazolyl, isoxazolyl, isothiazolyl and oxathiazolyl.

In a preferred embodiment of Formula (II),
R¹ is a C₁₋₁₂alkyl group which is optionally substituted with one or more substituents being selected from -OH, a halogen atom (e.g. F, CI), -NH₂, -NH(C₁₋₆alkyl) and -N(C₁₋₆alkyl)₂,
R⁴, R⁷, and R⁹ are selected independently from each other from hydrogen, an aryl group (e.g. phenyl), a heteroaryl group, C₁₋₆alkyl and -O-(C₁₋₆alkyl), the aryl group and/or the heteroaryl group optionally being substituted with one or more substituents which are selected independently from each other from C₁₋₆alkyl, C₁₋₆haloalkyl (e.g. C₁₋₆fluoroalkyl) and -O-(C₁₋₆alkyl),
R², R³, R⁵, R⁶, R⁸, R¹⁰, and R¹¹ are selected independently from each other from hydrogen and a C₁₋₆alkyl group.

Exemplary compounds of Formula (II) are those of the following Formulas (11-1), (II-2) and (II-3):

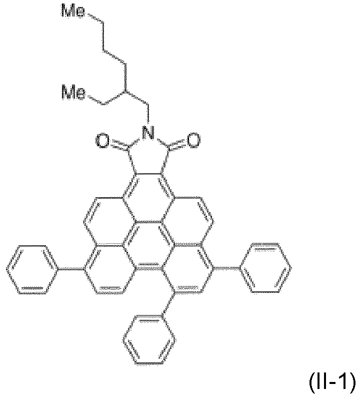

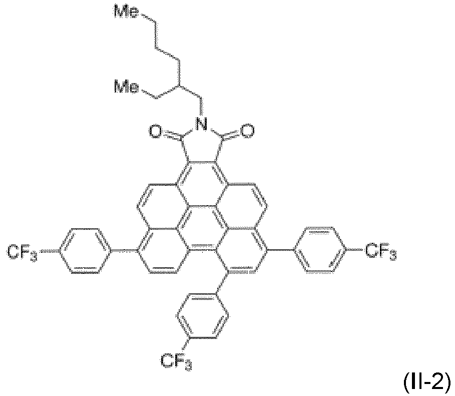

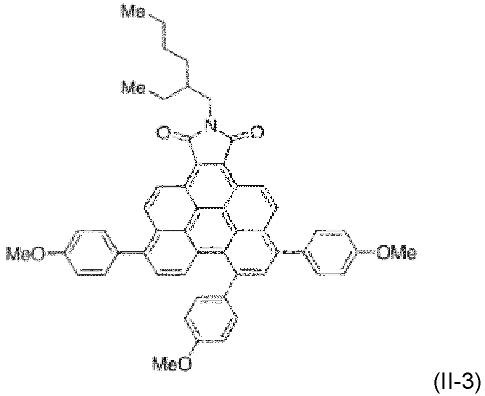

As outlined above, the polycyclic compound can be a compound of Formula (III).

The compound of Formula (III) might be associated with one or more charge-compensating anions, e.g. one or more weakly coordinating anions such as [BF₄]⁻, [ClO₄]⁻, [SbF₆]⁻ and [PF₆]⁻. However, other anions might be present as well.

In a preferred embodiment of Formula (III),
R¹ is a C₁₋₆alkyl group,
R¹⁰ is a phenyl group which is optionally substituted with one or more substituents being selected independently from each other from C₁₋₆alkyl, C₁₋₆haloalkyl (e.g. C₁₋₆fluoroalkyl) and -O-(C₁₋₆alkyl),
R² to R⁹ are selected independently from each other from hydrogen and a C₁₋₆alkyl group.

An exemplary compound of Formula (III) has the following Formula (III-1):

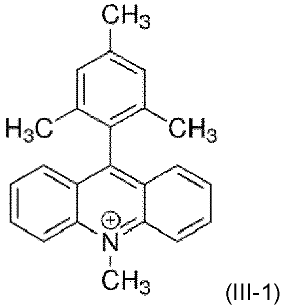

As already outlined above, in addition to the polycyclic compound of Formula (I), (II) or (III), the composition of the present invention comprises a palladium compound which is selected from a palladium salt or a palladium complex or a mixture thereof.

Preferably, the palladium compound is a catalyst or precatalyst for a Suzuki cross-coupling reaction. Palladium compounds which may act as a catalyst or precatalyst for Suzuki cross-coupling reactions are known to the skilled person and are commercially available or can be prepared by commonly known preparation methods.

If the palladium compound is a salt, it is preferably a palladium acetate, a palladium acetylacetonate or a palladium halide (such as palladium chloride (PdCl₂), palladium bromide (PdBr₂), or palladium iodide (PdI₂)), or any mixture thereof.

The composition may additionally comprise (iii) a phosphine or a bipyridine (in particular a 2,2'-bipyridine) compound, in particular if the palladium compound is a salt (such as a palladium acetate, a palladium acetylacetonate or a palladium halide). The phosphine or bipyridine compound and the palladium compound (in particular the palladium salt) might be kept in separate containers and brought into contact when performing the Suzuki cross-coupling reaction, thereby forming *in situ* a palladium complex which comprises one or more phosphine ligands.

The phosphine compound which is optionally present in the composition as component (iii) might be a tri-C₁₋₆alkyl phosphine, a tri-C₅₋₇cycloalkyl phosphine (preferably a tricyclohexyl phosphine), a triarylphosphine (in particular a triphenyl phosphine) wherein each of the aryl groups (which are preferably phenyl groups) is optionally substituted with one or more C₁₋₄haloalkyl (e.g. -CF₃), C₁₋₄alkyl (e.g. methyl) or C₁₋₄alkoxy (e.g. methoxy).

Exemplary phosphine compounds are those of the following Formulas (XII-1), (XII-2), and (XII-3):

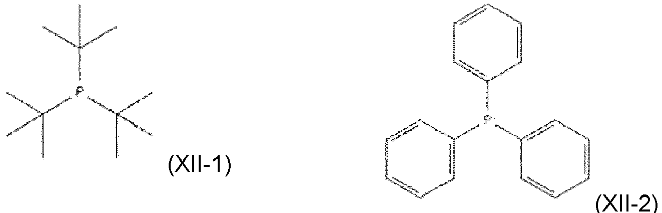

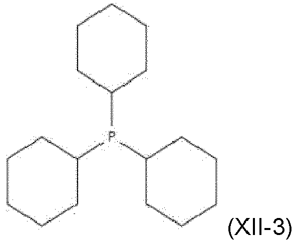

According to another exemplary embodiment, the phosphine compound is of Formula (XIII):

P(R¹)(R²)(R³) (XIII)

wherein
R¹ and R² are, independently from each other, selected from C₁₋₆alkyl and C₅₋₇cycloalkyl (e.g. cyclohexyl),
R³ is biphenyl which is optionally substituted with one or more R⁴,
R⁴, if present, is C₁₋₆alkyl, C₁₋₆alkoxy, phenyl, or pyridyl.

Exemplary phosphine compounds of Formula (XIII) may have one of the following Formulas (XIII-1) to (XIII-4):

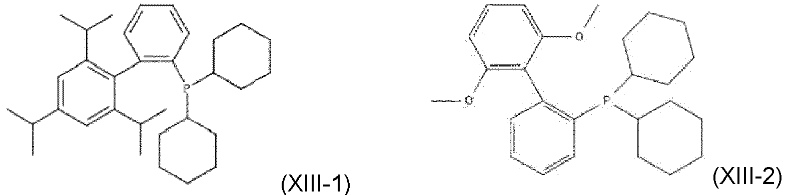

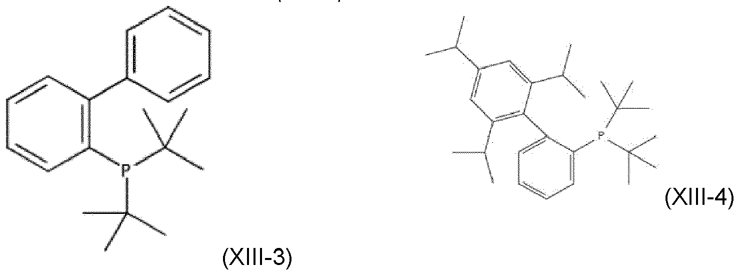

According to another exemplary embodiment, the phosphine compound is a ferrocenyl diphosphine or a xanthenyl diphosphine, which may have one of the following Formulas (XIV-1), (XIV-2) and (XV-1):

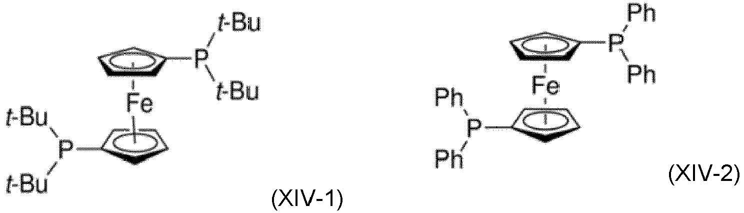

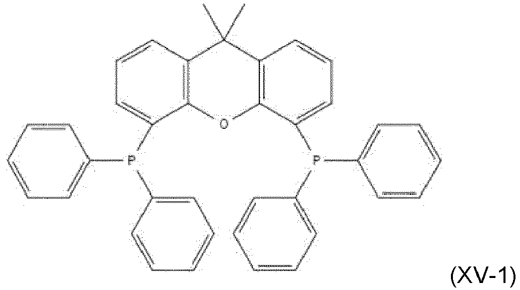

According to an exemplary embodiment of the present invention, the composition comprises (i) a palladium salt (such as a palladium acetate, a palladium acetylacetonate or a palladium halide); (ii) a polycyclic compound of Formula (Ia); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (Ia) have been described above.

According to another exemplary embodiment of the present invention, the composition comprises (i) a palladium salt (such as a palladium acetate, a palladium acetylacetonate or a palladium halide); (ii) a polycyclic compound of Formula (Ib); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (Ib) have been described above.

According to another exemplary embodiment of the present invention, the composition comprises (i) a palladium salt (such as a palladium acetate, a palladium acetylacetonate or a palladium halide); (ii) a polycyclic compound of Formula (Ic); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (Ic) have been described above.

According to another exemplary embodiment of the present invention, the composition comprises (i) a palladium salt (such as a palladium acetate, a palladium acetylacetonate or a palladium halide); (ii) a polycyclic compound of Formula (Id); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (Id) have been described above.

According to another exemplary embodiment of the present invention, the composition comprises (i) a palladium salt (such as a palladium acetate, a palladium acetylacetonate or a palladium halide); (ii) a polycyclic compound of Formula (II); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (II) have been described above.

According to an exemplary embodiment of the present invention, the composition comprises (i) a palladium salt (such as a palladium acetate, a palladium acetylacetonate or a palladium halide); (ii) a polycyclic compound of Formula (III); and optionally (iii) a phosphine compound (such as a trialkyl phosphine, a triaryl phosphine, a phosphine of Formula XIII, a ferrocenyl diphosphine or a xanthenyl diphosphine). Preferred embodiments of the polycyclic compound of Formula (III) have been described above.

If the palladium compound is a palladium complex, it preferably comprises one or more ligands which are selected from a phosphine, an N-heterocyclic carbene, an amine, or any combination thereof. In addition to these ligands, the palladium complex may comprise other ligands as well.

In an exemplary embodiment of the present invention, the palladium complex is of Formula (IV) 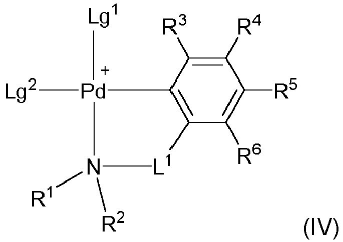 wherein
L¹ is a bivalent linker,
R¹ and R² are selected independently from each other from hydrogen and a C₁₋₄alkyl group, or
R¹ and R² together with the nitrogen atom to which they are attached form a five- or six-membered ring (which might be an aromatic or non-aromatic ring),
R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and the remaining residues (i.e. those of the residues R³ to R⁶ which do not form a ring) are selected independently from each other from hydrogen and C₁₋₄alkyl;
Lg¹ and Lg² are, independently from each other, a non-ionic ligand.

The compound of Formula (IV) might be associated with one or more charge-compensating anions, e.g. one or more weakly coordinating anions such as [BF₄]⁻, [ClO₄]⁻, [SbF₆]⁻ and [PF₆]⁻. However, other anions might be present as well.

Preferably, ligand Lg² is an N-heterocyclic carbene; and/or ligand Lg¹ is a pyridine, a pyrrole, an imidazole, a pyrazole or a pyrimidine, each of which is optionally substituted with one or more halogens.

According to an exemplary embodiment of Formula (IV),
L¹ is a C₁₋₃ alkylene group (e.g. -CH₂-; -CH₂-CH₂-; -CH₂-CH₂-CH₂-),
Lg¹ is a pyridine, a pyrrole, an imidazole, a pyrazole or a pyrimidine, each of which is optionally substituted with one or more halogens,
Lg² is an N-heterocyclic carbene.

The N-heterocyclic carbene ligand Lg² can be of Formula (V) or Formula (VI): 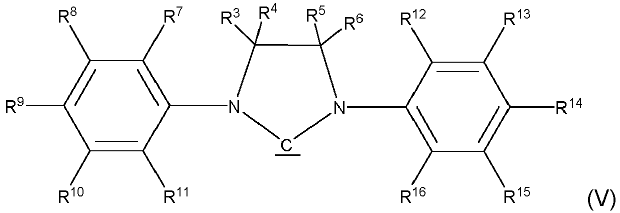 wherein in Formula (V)
R³ to R¹⁶ are selected independently from each other from hydrogen and C₁₋₄alkyl; or at least two of the residues R³ to R¹⁶ which are adjacent to each other form together with the carbon atoms to which they are attached a 5- or 6-membered ring while the remaining residues (i.e. those of the residues R³ to R¹⁶ not forming a ring) are selected independently from each other from hydrogen and C₁₋₄alkyl; 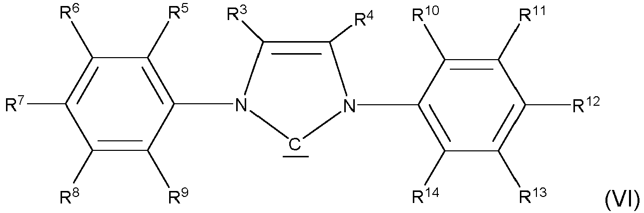 wherein in Formula (VI)
R³ to R¹⁴ are selected independently from each other from hydrogen and C₁₋₄ alkyl; or at least two of the residues R³ to R¹⁴ which are adjacent to each other form together with the carbon atoms to which they are attached a 5- or 6-membered ring while the remaining residues (i.e. those of the residues R³ to R¹⁴ not forming a ring) are selected independently from each other from hydrogen and C₁₋₄alkyl.

An exemplary palladium complex of Formula (IV) has the following Formula (IV-1):

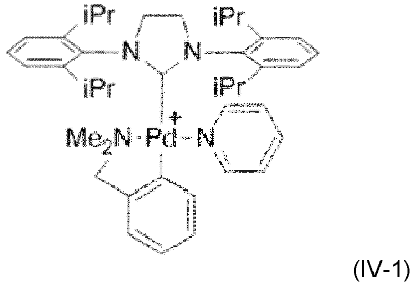

According to an exemplary embodiment of the present invention, the composition comprises (i) a palladium complex of Formula (IV); and (ii) a polycyclic compound of Formula (Ia). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound t of Formula (Ia) have been described above.

According to another exemplary embodiment of the present invention, the composition comprises (i) a palladium complex of Formula (IV); and (ii) a polycyclic compound of Formula (Ib). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound of Formula (Ib) have been described above.

According to another exemplary embodiment of the present invention, the composition comprises (i) a palladium complex of Formula (IV); and (ii) a polycyclic compound of Formula (Ic). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound of Formula (Ic) have been described above.

According to another exemplary embodiment of the present invention, the composition comprises (i) a palladium complex of Formula (IV); and (ii) a polycyclic compound of Formula (Id). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound of Formula (Id) have been described above.

According to another exemplary embodiment of the present invention, the composition comprises (i) a palladium complex of Formula (IV); and (ii) a polycyclic compound of Formula (II). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound of Formula (II) have been described above.

According to another exemplary embodiment of the present invention, the composition comprises (i) a palladium complex of Formula (IV); and (ii) a polycyclic compound of Formula (III). Preferably, ligand Lg² in Formula (IV) is a N-heterocyclic carbene (e.g. of Formula (V) or (VI)); and/or ligand Lg¹ is a pyridine, a pyrrole, an imidazole, a pyrazole or a pyrimidine, each of which is optionally substituted with one or more halogens. Preferred embodiments of the polycyclic compound of Formula (III) have been described above.

The palladium complex being present in the composition of the present invention can be of Formula (VII) 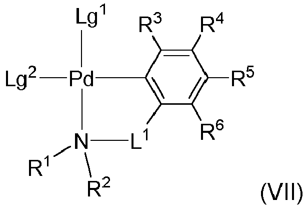 wherein
L¹ is a bivalent linker,
R¹ and R² are selected independently from each other from hydrogen and a C₁₋₄ alkyl group, or
R¹ and R² together with the nitrogen atom to which they are attached form a five- or six-membered ring,
R³, R⁴, R⁵, and R⁶ are selected independently from each other from hydrogen and C₁₋₄ alkyl, or at least two of the residues R³ to R⁶ which are adjacent to each other form together with the carbon atoms to which they are attached a five- or six-membered aromatic ring and the remaining residues (i.e. those of the residues R³ to R⁶ which do not form a ring) are selected independently from each other from hydrogen and C₁₋₄alkyl,
Lg¹ is a non-ionic ligand,
Lg² is an anionic ligand.

Preferably, the non-ionic ligand Lg¹ in Formula (VII) is a phosphine.

Preferably, the anionic ligand Lg² in Formula (VII) is a weakly or non-coordinating anionic ligand, such as an alkyl sulfonate (e.g. mesylate) or an aryl sulfonate (e.g. tosylate). However, other anionic ligands might be used as well.

In an exemplary embodiment of Formula (VII),
L¹ is a phenylene (in particular a 1,2-phenylene) group or a C₁₋₃alkylene group (e.g. -CH₂-; - CH₂-CH₂-; - CH₂-CH₂-CH₂-);
the non-ionic ligand Lg¹ is a phosphine,
the anionic ligand Lg² is an alkyl sulfonate (e.g. mesylate) or an aryl sulfonate (e.g. tosylate).

The phosphine ligand Lg¹ in Formula (VII) can be phosphine of Formula (VIII):

P(R¹)(R²)(R³) (VIII)

wherein
R¹ and R² are selected independently from each other from C₁₋₆alkyl, C₅₋₇cycloalkyl (in particular cyclohexyl), and phenyl which is optionally substituted with one or more C₁₋₄ alkyl groups,
R³ is biphenyl or binaphthyl, each of which is optionally substituted with one or more R⁴,
R⁴, if present, is C₁₋₆alkyl, -NH₂, -N(H)(C₁₋₆alkyl), -N(C₁₋₆alkyl)₂, -P(Phenyl)₂, or OC₁₋₆alkyl.

Exemplary palladium complexes of Formula (VII) can have one of the following Formulas (VII-1) to Formula (VII-7):

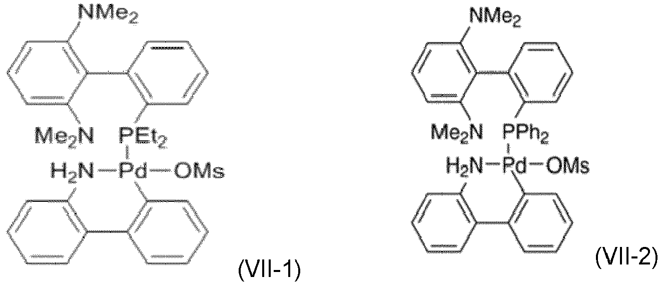

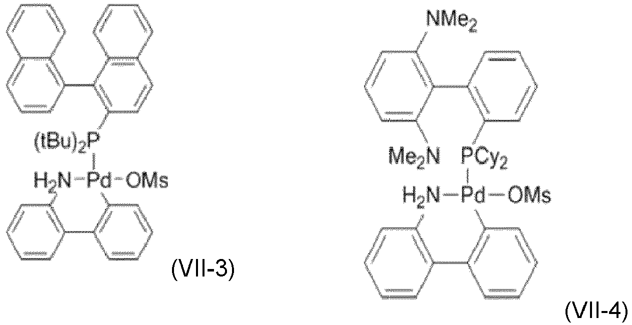

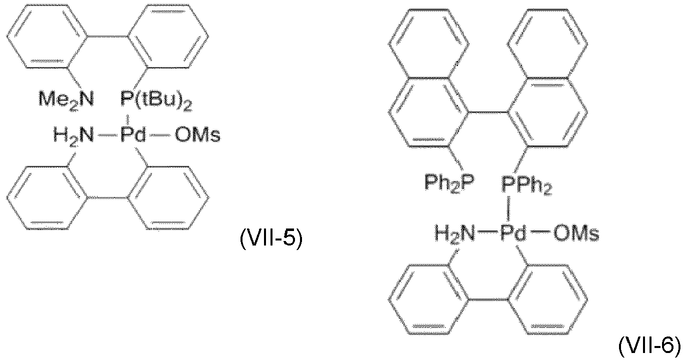

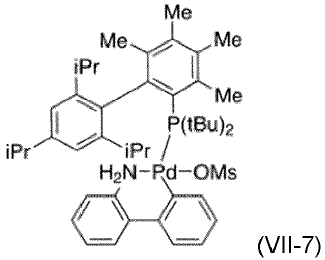

According to another exemplary embodiment, the phosphine ligand Lg¹ in Formula (VII) is a ferrocenyl phosphine.

Exemplary palladium complexes of Formula (VII) containing a ferrocenyl phosphine ligand are those of Formula (VII-8) or Formula (VII-9):

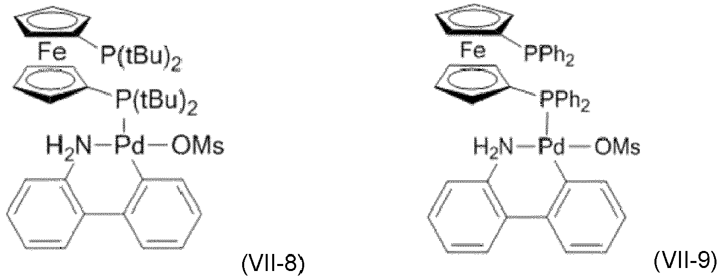

According to another exemplary embodiment, the phosphine ligand Lg¹ in Formula (VII) is a trialkyl phosphine (e.g. P(C₁₋₆ alkyl)₃). An exemplary palladium complex of Formula (VII) containing a trialkyl phosphine ligand is the palladium complex of Formula (VII-10):

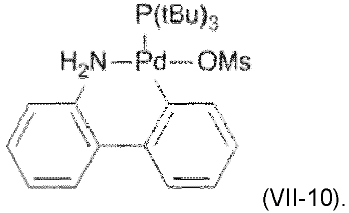

Other exemplary palladium complexes of Formula (VII) containing a phosphine ligand Lg¹ are those of Formula (VII-11), (VII-12), or (VII-13):

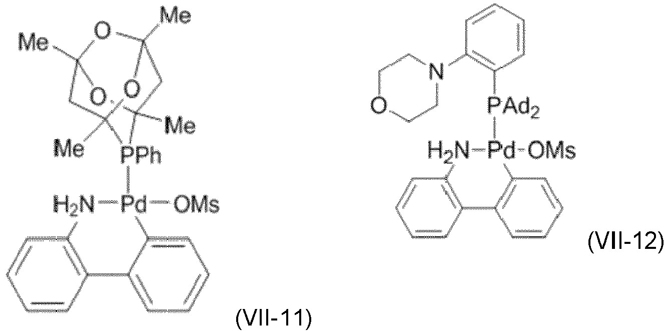

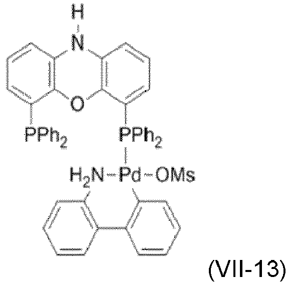

According to an exemplary embodiment of the present invention, the composition comprises (i) a palladium complex of Formula (VII); and (ii) a polycyclic compound of Formula (Ia). Preferably, the non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (Ia) have been described above.

According to another exemplary embodiment of the present invention, the composition comprises (i) a palladium complex of Formula (VII); and (ii) a polycyclic compound of Formula (Ib). Preferably, the non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (Ib) have been described above.

According to another exemplary embodiment of the present invention, the composition comprises (i) a palladium complex of Formula (VII); and (ii) a polycyclic compound of Formula (Ic). Preferably, non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (Ic) have been described above.

According to another exemplary embodiment of the present invention, the composition comprises (i) a palladium complex of Formula (VII); and (ii) a polycyclic compound of Formula (Id). Preferably, the non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (Id) have been described above.

According to another exemplary embodiment of the present invention, the composition comprises (i) a palladium complex of Formula (VII); and (ii) a polycyclic compound of Formula (II). Preferably, non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (II) have been described above.

According to another exemplary embodiment of the present invention, the composition comprises (i) a palladium complex of Formula (VII); and (ii) a polycyclic compound of Formula (III). Preferably, non-ionic ligand Lg¹ in Formula (VII) is a phosphine (e.g. a phosphine of Formula (VIII), a ferrocenyl phosphine or a phosphine in one of the Formulas (VII-11) to (VII-13)). Preferred embodiments of the polycyclic compound of Formula (III) have been described above.

The palladium complex being present in the composition of the present invention can be of Formula (IX):

Pd(L)₃₋₄ (IX)

wherein
the ligands L are, independently from each other, a triaryl phosphine (preferably a triphenyl phosphine), wherein each aryl group (which preferably is a phenyl group) is optionally substituted with one or more R¹,
R¹, if present, is C₁₋₄haloalkyl (e.g. -CF₃), C₁₋₄alkyl (e.g. methyl) or C₁₋₄alkoxy (e.g. methoxy).

Exemplary palladium complexes of Formula (IX) are those of Formula (IX-1) or (IX-2):

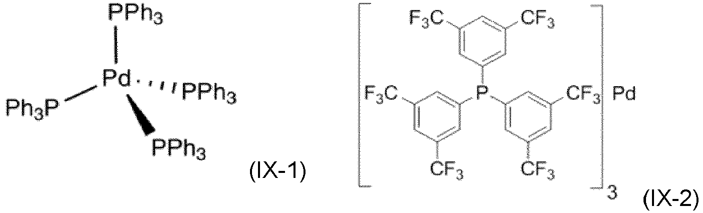

According to an exemplary embodiment of the present invention, the composition comprises (i) a palladium complex of Formula (IX); and (ii) a polycyclic compound of Formula (Ia). Preferred embodiments of the polycyclic compound of Formula (Ia) have been described above.

According to another exemplary embodiment of the present invention, the composition comprises (i) a palladium complex of Formula (IX); and (ii) a polycyclic compound of Formula (Ib). Preferred embodiments of the polycyclic compound of Formula (Ib) have been described above.

According to another exemplary embodiment of the present invention, the composition comprises (i) a palladium complex of Formula (IX); and (ii) a polycyclic compound of Formula (Ic). Preferred embodiments of the polycyclic compound of Formula (Ic) have been described above.

According to another exemplary embodiment of the present invention, the composition comprises (i) a palladium complex of Formula (IX); and (ii) a polycyclic compound of Formula (Id). Preferred embodiments of the polycyclic compound of Formula (Id) have been described above.

According to another exemplary embodiment of the present invention, the composition comprises (i) a palladium complex of Formula (IX); and (ii) a polycyclic compound of Formula (II). Preferred embodiments of the polycyclic compound of Formula (II) have been described above.

According to another exemplary embodiment of the present invention, the composition comprises (i) a palladium complex of Formula (IX); and (ii) a polycyclic compound of Formula (III). Preferred embodiments of the polycyclic compound of Formula (III) have been described above.

The palladium complex being present in the composition of the present invention can be of Formula (X): 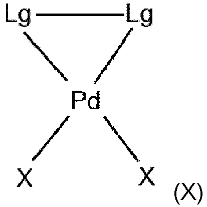 wherein
Lg-Lg is a chelating ferrocenyl diphosphine ligand,
X is a halide (e.g. CI).

Exemplary palladium complexes of Formula (X) are those of Formula (X-1) or (X-2):

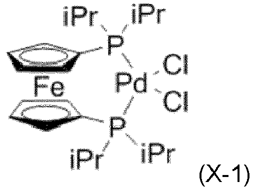

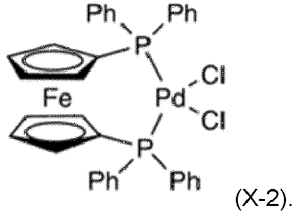

According to an exemplary embodiment of the present invention, the composition comprises (i) a palladium complex of Formula (X); and (ii) a polycyclic compound of Formula (Ia). Preferred embodiments of the polycyclic compound of Formula (Ia) have been described above.

According to another exemplary embodiment of the present invention, the composition comprises (i) a palladium complex of Formula (X); and (ii) a polycyclic compound of Formula (Ib). Preferred embodiments of the polycyclic compound of Formula (Ib) have been described above.

According to another exemplary embodiment of the present invention, the composition comprises (i) a palladium complex of Formula (X); and (ii) a polycyclic compound of Formula (Ic). Preferred embodiments of the polycyclic compound of Formula (Ic) have been described above.

According to another exemplary embodiment of the present invention, the composition comprises (i) a palladium complex of Formula (X); and (ii) a polycyclic compound of Formula (Id). Preferred embodiments of the polycyclic compound of Formula (Id) have been described above.

According to another exemplary embodiment of the present invention, the composition comprises (i) a palladium complex of Formula (X); and (ii) a polycyclic compound of Formula (II). Preferred embodiments of the polycyclic compound of Formula (II) have been described above.

According to another exemplary embodiment of the present invention, the composition comprises (i) a palladium complex of Formula (X); and (ii) a polycyclic compound of Formula (III). Preferred embodiments of the polycyclic compound of Formula (III) have been described above.

The composition may contain the palladium compound and the polycyclic compound of Formula (I), (II) or (III) in the form of a mixture. Optionally, one or more additional components might be present in the mixture. Alternatively, the mixture may consist of the palladium compound and the polycyclic compound of Formula (I), (II) or (III).

The molar ratio between the palladium compound and the polycyclic compound of Formula (I), (II) or (III) might be in the range of from 100/1 to 1/100, more preferably 10/1 to 1/10 or even more preferably 2/1 to 1/2.

Instead of comprising a mixture of the palladium compound and the polycyclic compound of Formula (I), (II) or (III), the composition can be a kit which provides the palladium compound and the polycyclic compound of Formula (I), (II) or (III) in separate containers.

The present invention also relates to a method of performing a Suzuki cross-coupling reaction, wherein a first reactant is reacted with a second reactant under irradiation of light in the presence of the composition of the present invention, the first reactant comprising an aromatic or N-heteroaromatic ring to which a boronic acid or boronic acid ester group is attached, and the second reactant comprising an aromatic or N-heteroaromatic ring to which a halogen (CI, Br, I), a pseudohalogen (cyano, cyanate, isocyanate), or a sulfonate is attached.

*"Under irradiation of light"* refers to light provided by an external light source (e.g. lamps such as an UV lamp or an UV/VIS lamp, one or more LEDs, a laser, etc.).

The light may have a wavelength in the range of from 254 nm to 800 nm, preferably 360 nm to 550 nm. The light can be a monochromatic light; or may cover a continuous wavelength spectrum (e.g. in the UV and/or visible spectrum). The light reaching the reaction medium may have an irradiance of 10 to 2000 milliwatts per square centimeter. However, lower or higher irradiance values can be used as well.

The first and second reactants might be reacted in the presence of a base which is preferably selected from carbonate (e.g. an alkali metal carbonate), a hydroxide (e.g. an alkali metal hydroxide), a phosphate (e.g. an alkali metal phosphate), an alkoxide (e.g. an alkali metal alkoholate). Typically, the Suzuki cross-coupling reaction is carried out in a solvent which is preferably selected from water, an aromatic solvent, an ether, a glycol ether, dimethyl formamide, acetonitrile, an alcohol, or a mixture of two or more of these solvents. The reactants might be reacted at a temperature of from 20 °C to 70 °C.

Furthermore, the present invention relates to the use of the composition described above as a catalyst system for a light-assisted Suzuki carbon-carbon cross-coupling reaction. As known to the skilled person, *"light assisted"* means that the reaction medium in which the Suzuki carbon-carbon cross-coupling reaction is carried out is irradiated by an external light source (e.g. lamps such as UV or UV/VIS lamps, LEDs, laser, etc.).

The features disclosed in the claims, the specification, and the drawings maybe essential for different embodiments of the claimed invention, both separately and in any combination with each other.

### Examples

In Comparative Example CE1 and Inventive Examples IE1-IE2, the same reactants (4-trifluoromethyl phenyl boronic acid pinacol ester (CAS No. 214360-65-3), i.e. an organoboron compound, and 3-chloropyridine, i.e. a heteroaryl halide) were used. Furthermore, in CE1, IE1 and IE2, the same palladium complex was present in the reaction medium. However, in IE1 and IE2, the reaction medium additionally contained a polycyclic compound and was irradiated by light from an external light source.

### Comparative Example CE1 (no polycyclic compound of Formula (I), (II) or (III); no light irradiation of the reaction medium):

In the glovebox, 65.3 mg of 4-trifluoromethyl phenyl boronic acid pinacol ester (CAS No. 214360-65-3) was added to a vial and dissolved in 1280 µl of acetonitrile, followed by 100 µl of a 0.04 M solution of [1,3-bis(2,6-di-*iso*-propylphenyl)-4,5-dihydroimidazol-2-ylidene]{2-[(dimethylamino-kN)methyl]phenyl-kC}(pyridine)palladium(II) tetrafluoroborate palladacycle (i.e. a palladium complex) in DMF. The palladium complex is commercially available (e.g. from Strem Chemicals). An amber vial was used to preclude light from coming in. Accordingly, no irradiation of the reaction medium took place. In the glove box, 19 µl of neat degassed 3-chloropyridine (i.e. a heteroaryl halide) was added to the vial, followed by 400 µl of a degassed aqueous 0.85 M solution of K₃PO₄. Finally, to this vial (vial 1), 100 µl of degassed benzene was added.

The reactants, the catalyst system and the reaction conditions used in Comparative Example CE1 are shown below in Table 1:

**Table 1: Reactants, catalyst system and reaction conditions used in CE1**

| | | |
|---|---|---|
| Reactants | Organoboron compound | |
| | Heteroaryl halide | 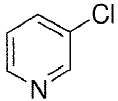 |
| Catalyst system | Pd complex | 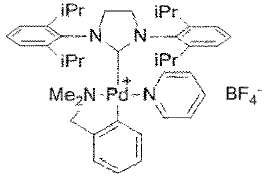 |
| | Polycyclic compound of Formula (I), (II) or (III) | No |
| Reaction temperature | | 40°C |
| Irradiation of reaction medium | | No |

The total volume of this reaction was 2.4 ml (2.0 ml organic, and 400 µl aqueous), with 2 mol% palladium complex.

The vial was placed in a glycol/ water bath which was set to 40 °C, and illuminated with 445 nm by an array of 96 LEDs, with stirring for 5 hours. However, as already mentioned above, an amber vial was used in Comparative Example CE1, thereby precluding essentially all the light from reaching the reaction medium.

After 5 hours, the vial was allowed to cool to room temperature before being opened. Then 1 equivalent of trifluorotoluene was added as an internal ¹⁹F-NMR standard, and a 20 µl sample was taken for TLC (Thin Layer Chromatography). A further 20 µL was taken for ¹⁹F-NMR, to determine conversion of starting material to product.

By ¹⁹F-NMR, 0% product conversion was obtained. No Suzuki cross-coupling product was obtained.

### Inventive Example IE1

In the glovebox, 65.3 mg of 4-trifluoromethyl phenyl boronic acid pinacol ester (CAS No. 214360-65-3), i.e. the same organoboron reactant as used in Comparative Example CE1, was added to a clear-glass vial and dissolved in 1280 µl of acetonitrile, followed by 100 µl of a 0.04 M solution of [1,3-Bis(2,6-di-i-propylphenyl)-4,5-dihydroimidazol-2-ylidene]{2-[(dimethylamino-kN)methyl]phenyl-kC}(pyridine)palladium(II) tetrafluoroborate palladacycle (i.e. the same Pd complex as used in Comparative Example CE1) in DMFIn the glove box, 19 µl of neat degassed 3-chloropyridine (i.e. the same heteroaryl halide reactant as used in Comparative Example CE1) was added to each clear-glass vial, followed by 400 µl of a degassed aqueous 0.85 M solution of K₃PO₄. Finally, to this clear-glass vial, 100 µl of a solution (0.02 M) of the polycyclic compound of Formula (I-2) in degassed benzene was added.

The reactants, the catalyst system and the reaction conditions used in Inventive Example IE1 are shown below in Table 2:

**Table 2: Reactants, catalyst system and reaction conditions used in IE1**

| | | |
|---|---|---|
| Reactants | Organoboron compound | |
| | Heteroaryl halide | 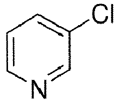 |
| Catalyst system | Pd complex | 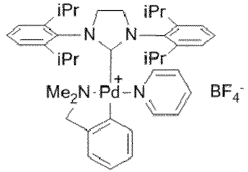 |
| | Polycyclic compound of Formula (I), (II) or (III) | 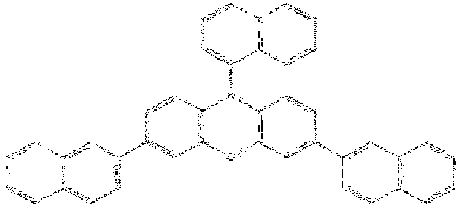 |
| Reaction temperature | | 40°C |
| Irradiation of reaction medium | | Yes (at 445 nm by an array of LEDs) |

The total volume of this reaction was 2.4 ml (2.0 ml organic, and 400 µl aqueous), with 2 mol% palladium complex, and 1 mol% polycyclic compound.

The clear-glass vial was placed in a glycol/ water bath which was set to 40 °C, and illuminated with 445 nm by an array of 96 LEDs, with stirring for 5 hours. After 5 hours, the clear-glass vial was allowed to cool to room temperature before being opened. Then 1 equivalent of trifluorotoluene was added as an internal ¹⁹F-NMR standard, and a 20 µl sample was taken for TLC. A further 20 µl was taken from the vial for ¹⁹F-NMR, to determine conversion of starting material to product.

By ¹⁹F-NMR, 60% Suzuki coupling product conversion was observed.

### Inventive Example IE2

In the glovebox, 65.3 mg of the 4-trifluoromethyl phenyl boronic acid pinacol ester (CAS No. 214360-65-3), i.e. the same organoboron reactant as used in Comparative Example CE1, was added to a clear-glass vial and dissolved in 1280 µl of acetonitrile, followed by 100 µl of a 0.04 M solution of the [1,3-Bis(2,6-di-i-propylphenyl)-4,5-dihydroimidazol-2-ylidene]{2-[(dimethylamino-kN)methyl]phenyl-kC}(pyridine)palladium(II) tetrafluoroborate palladacycle (i.e. the same Pd complex as used in Comparative Example CE1) in DMF. In the glove box, 19 µl of neat degassed 3-chloropyridine (i.e. the same heteroaryl halide reactant as used in Comparative Example CE1) was added to each vial, followed by 400 µl of a degassed aqueous 0.85 M solution of K₃PO₄. Finally, to this vial, 100 µl of a solution (0.02 M) of polycyclic compound of Formula (I-3) in degassed benzene was added.

The reactants, the catalyst system and the reaction conditions used in Inventive Example IE2 are shown below in Table 3:

**Table 3: Reactants and catalyst system used in IE2**

| | | |
|---|---|---|
| Reactants | Organoboron compound | |
| | Heteroaryl halide | 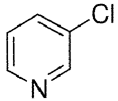 |
| Catalyst system | Pd complex | 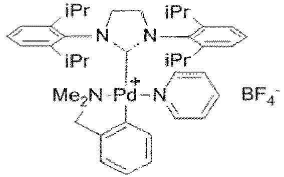 |
| | Polycyclic compound of Formula (I), (II) or (III) | 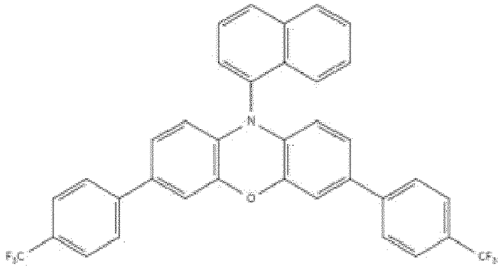 |
| Reaction temperature | | 40°C |
| Irradiation of reaction medium | | Yes (at 445 nm by an array of LEDs) |

The total volume of this reaction was 2.4 ml (2.0 ml organic, and 400 µl aqueous), with 2 mol% palladium complex, and 1 mol% polycyclic compound.

The vial was placed in a glycol/ water bath which was set to 40 °C, and illuminated with 445 nm by an array of 96 LEDs, with stirring for 5 hours. After 5 hours, the vial was allowed to cool to room temperature before being opened. Then 1 equivalent of trifluorotoluene was added as an internal ¹⁹F-NMR standard, and a 20 µl sample was taken for TLC. A further 20 µl was taken for ¹⁹F-NMR, to determine conversion of starting material to product.

By ¹⁹F-NMR, 67% Suzuki coupling product conversion was observed.

Suzuki cross-coupling product yields (determined by ¹⁹F NMR) in Comparative Example CE1 and Inventive Examples E1-E2 are summarized below in Table 4.

**Table 4: Yield of Suzuki cross-coupling reaction product in CE1, IE1 and IE2**

| | Reaction temperature [°C] | Yield of Suzuki cross-coupling reaction product [%], determined by 19F NMR |
|---|---|---|
| CE1 | 40 | 0 |
| IE1 | 40 | 60 |
| IE2 | 40 | 67 |

As demonstrated by the examples, an efficient light-assisted Suzuki cross-coupling reaction between an organoboron compound and a (hetero-)aryl halide is accomplished even at low reaction temperature if a combination of a palladium compound and a polycyclic compound of Formula (I), (II) or (III) is used as a catalyst system.

As described above, the present invention relates to the following embodiments {01} to {51}:
Embodiment {01} relates to a composition, comprising
   (i) a palladium compound which is selected from a palladium salt or a palladium complex or a mixture thereof,
   (ii) a polycyclic compound of Formula (I), (II) or (III): 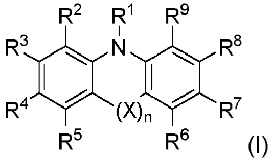 wherein
      n is 0 or 1,
      X is O, C(R¹⁰)(R¹¹), or N(R¹²),
      R¹ to R¹² are selected independently from each other from hydrogen, an aryl group and a C₁₋₁₀alkyl group, the aryl group and/or the C₁₋₁₀alkyl group optionally being substituted with one or more substituents R¹³,
      wherein the one or more substituents R¹³, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 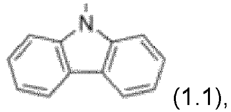 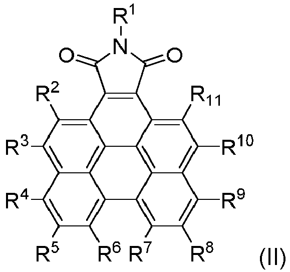 wherein
      R¹ is selected from a C₁₋₁₆alkyl group and an aryl group, the C₁₋₁₆alkyl group and/or the aryl group optionally being substituted with one or more substituents R¹², and/or the C₁₋₁₆alkyl group optionally being interrupted by one or more oxygen atoms;
      R² to R¹¹ are selected independently from each other from hydrogen, a C₁₋₁₆alkyl group, an aryl group, a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms; or
      at least two of the residues R² to R¹¹ which are adjacent to each other form together with the carbon atoms to which they are attached a five- or six-membered aromatic ring while the remaining residues (i.e. those of the residues R² to R¹¹ which do not form a ring) are selected independently from each other from hydrogen, a C₁₋₁₆alkyl group, an aryl group and a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms;
      wherein the one or more substituents R¹², if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl), 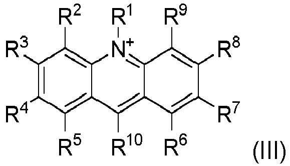 wherein
      R¹ is selected from a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
      R² to R⁹ are selected independently from each other from hydrogen, a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
      R¹⁰ is an aryl group which is optionally substituted with one or more substituents R¹², wherein the one or more substituents R¹¹, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl),
      wherein the one or more substituents R¹², if present, are selected independently from each other from a C₁₋₆alkyl group and an aryl group (e.g. a phenyl group), wherein the aryl group is optionally substituted with one or more C₁₋₆alkyl groups.
Embodiment {02} relates to the composition according to Embodiment {01}, wherein R¹ in Formula (I) is selected from a phenyl group, a naphthyl group and a biphenyl group, each of which is optionally substituted with one or more substituents R¹³, wherein R¹³ has the same meaning as in embodiment {01}.
Embodiment {03} relates to the composition according to Embodiment {01} or {02}, wherein in Formula (I)
   n is 1,
   X is O,
   R¹ is a naphthyl group (preferably a 1-naphthyl group) which is optionally substituted with one or more substituents R¹³,
   R², R³, R⁵, R⁶, R⁸ and R⁹ are selected independently from each other from hydrogen and a C₁₋₆alkyl group,
   R⁴ and R⁷ are selected independently from each other from a phenyl group, a biphenyl group and a naphthyl group, each of which is optionally substituted with one or more substituents R¹³, wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).
Embodiment {04} relates to the composition according to embodiment {03}, wherein the polycyclic compound is a compound of Formula (I-1), (I-2), (I-3) or (I-4): 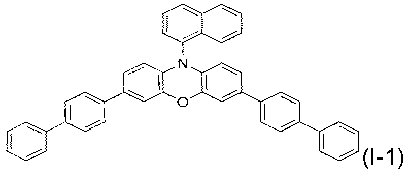 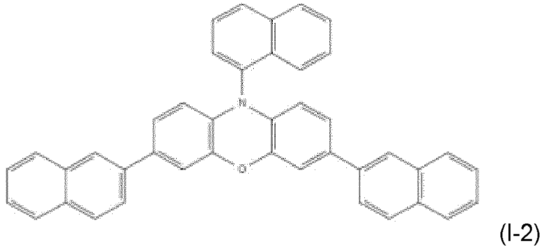 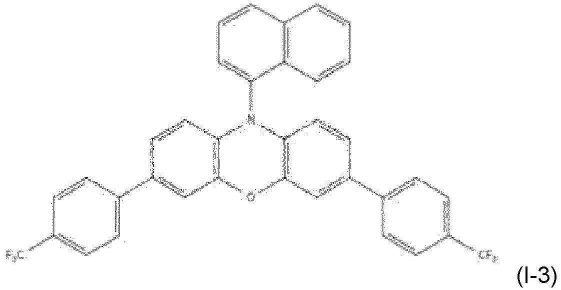 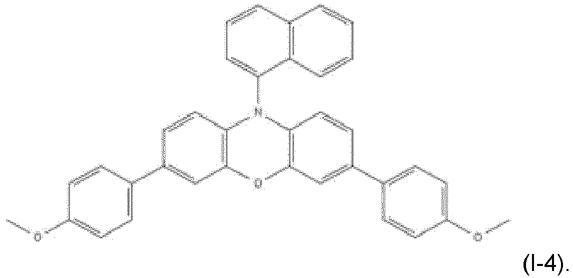
Embodiment {05} relates to the composition according to embodiment {01} or {02}, wherein in Formula (I)
   n is 1,
   X is C(R¹⁰)(R¹¹), wherein R¹⁰ and R¹¹ are selected independently from each other from hydrogen and a C₁₋₆alkyl group,
   R¹ is selected from a naphthyl group (preferably a 1-naphthyl or 2-naphthyl group), a phenyl group and a biphenyl group, each of which is optionally substituted with one or more substituents R¹³,
   R², R³, R⁵, R⁶, R⁸ and R⁹ are selected independently from each other from hydrogen and a C₁₋₆alkyl group,
   R⁴ and R⁷ are selected independently from each other from a phenyl group, a naphthyl group and a biphenyl group, each of which is optionally substituted with one or more substituents R¹³, wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).
Embodiment {06} relates to the composition according to embodiment {05}, wherein the polycyclic compound is a compound of one of the Formulas (I-5.1) to (I-5.7): 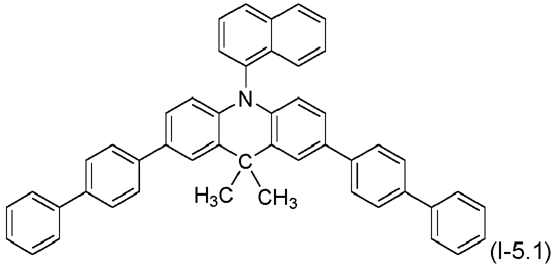 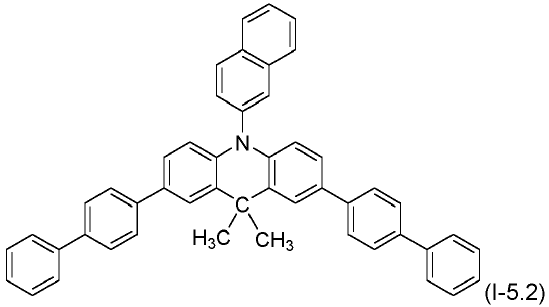 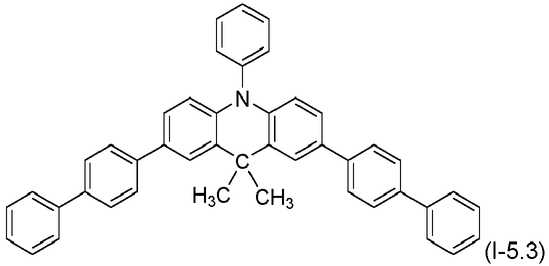 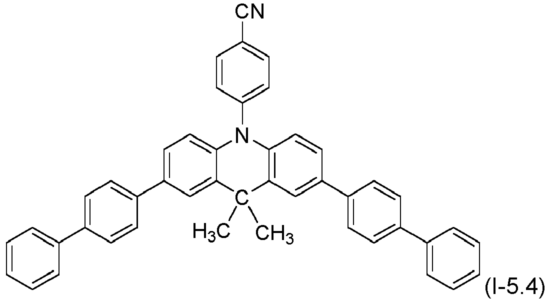 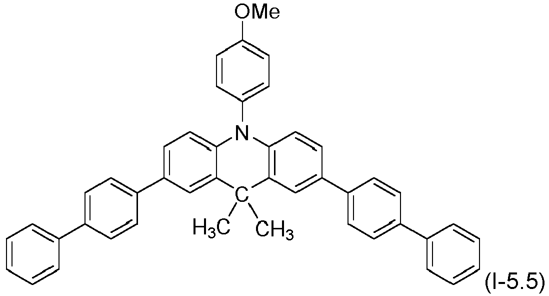 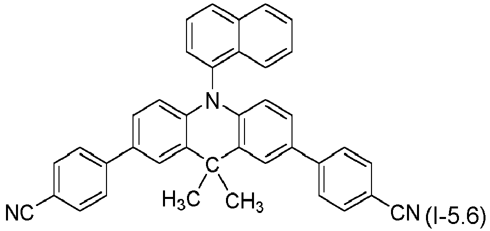 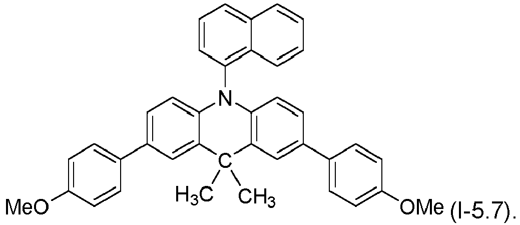
Embodiment {07} relates to the composition according to embodiments {01} or {02}, wherein in Formula (I)
   n is 1,
   X is N(R¹²),
   R¹ and R¹² are selected independently from each other from a phenyl group, a naphthyl group (such as a 2-naphthyl group or a 1 naphthyl group) and a biphenyl group, each of which is optionally substituted with one or more substituents R¹³,
   R² to R⁹ are selected independently from each other from hydrogen and a C₁₋₆alkyl group, or at least two, preferably all of the residues R³, R⁴, R⁷, and R⁸ are a phenyl group which is optionally substituted with one or more substituents R¹³ and the residues R², R⁵, R⁶ and R⁹ are selected independently from each other from hydrogen and a C₁₋₆alkyl group,
   wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).
Embodiment {08} relates to the composition according to embodiment {07}, wherein the polycyclic compound is a compound of Formula (I-6) or (I-7): 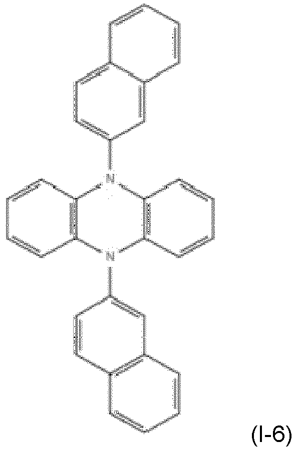 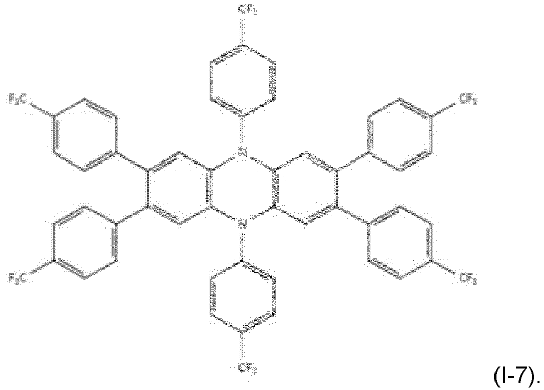
Embodiment {09} relates to the composition according to embodiment {01} or {02}, wherein in Formula (I)
   n is 0,
   R¹ is selected from a naphthyl (e.g. 1-naphthyl or 2-naphthyl) group, a phenyl group and a biphenyl group, each of which is optionally substituted with one or more R¹³,
   R² to R⁹ are selected independently from each other from hydrogen, a C₁₋₆alkyl group, a phenyl group, biphenyl group and a naphthyl group, the phenyl group, the biphenyl group and/or the naphthyl group optionally being substituted with one or more substituents R¹³,
   wherein, if present, the one or more substituents R¹³, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 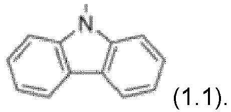
Embodiment {10} relates to the composition according to embodiment {09}, wherein the polycyclic compound is a compound of Formula (I-8): 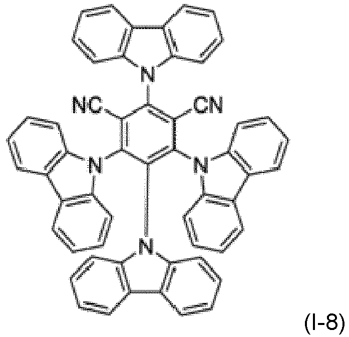
Embodiment {11} relates to the composition according to embodiment {01} or {02}, wherein in Formula (II)
   R¹ is a C₁₋₁₂alkyl group which is optionally substituted with one or more substituents being selected from -OH, a halogen atom (e.g. F, CI), -NH₂, -NH(C₁₋₆alkyl) and -N(C₁₋₆alkyl)₂,
   R⁴, R⁷, and R⁹ are selected independently from each other from hydrogen, an aryl group (e.g. phenyl), a heteroaryl group, C₁₋₆alkyl and -O-(C₁₋₆alkyl), the aryl group and/or the heteroaryl group optionally being substituted with one or more substituents which are selected independently from each other from C₁₋₆alkyl, C₁₋₆haloalkyl (e.g. C₁₋₆fluoroalkyl) and -O-(C₁₋₆alkyl),
   R², R³, R⁵, R⁶, R⁸, R¹⁰, and R¹¹ are selected independently from each other from hydrogen and a C₁₋₆alkyl group.
Embodiment {12} relates to the composition according to embodiment {11}, wherein the polycyclic compound is a compound of Formula (11-1), (II-2) or (II-3): 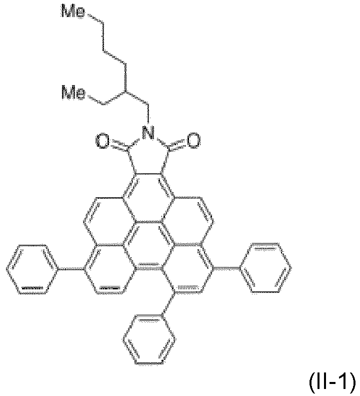 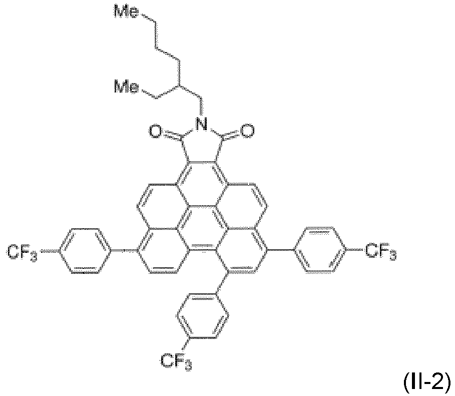 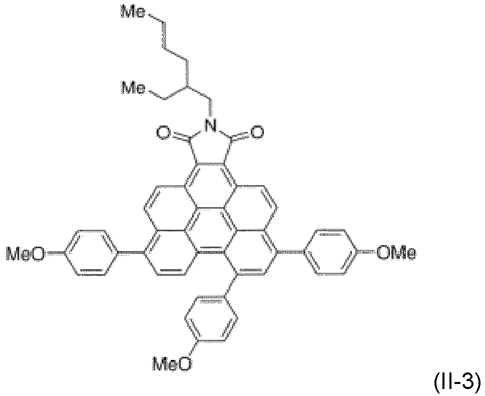
Embodiment {13} relates to the composition according to embodiment {01} or {02}, wherein in Formula (III)
   R¹ is a C₁₋₆alkyl group,
   R¹⁰ is a phenyl group which is optionally substituted with one or more substituents being selected independently from each other from C₁₋₆alkyl, C₁₋₆haloalkyl (e.g. C₁₋₆fluoroalkyl) and -O-(C₁₋₆alkyl),
   R² to R⁹ are selected independently from each other from hydrogen and a C₁₋₆alkyl group.
Embodiment {14} relates to the composition according to embodiment {13}, wherein the polycyclic compound is a compound of Formula (III-1): 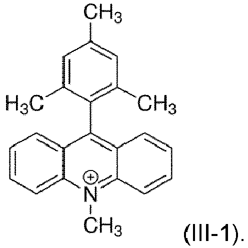
Embodiment {15} relates to the composition according to one of the embodiments {01} to {14}, wherein the palladium salt is a palladium acetate, a palladium acetylacetonate or a palladium halide (such as palladium chloride (PdCl₂), palladium bromide (PdBr₂), or palladium iodide (Pdl₂)), or any mixture thereof.
Embodiment {16} relates to the composition according to one of the embodiments {01} to {14}, wherein the palladium complex comprises one or more ligands which are selected from a phosphine, an N-heterocyclic carbene, an amine, or any combination thereof.
Embodiment {17} relates to the composition according to embodiment {16}, wherein the palladium complex is of Formula (IV) 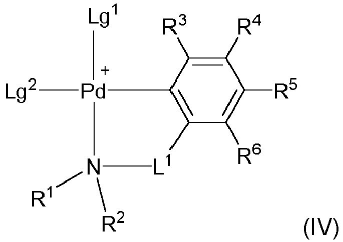 wherein
   L¹ is a bivalent linker,
   R¹ and R² are selected independently from each other from hydrogen and a C₁₋₄alkyl group, or
   R¹ and R² together with the nitrogen atom to which they are attached form a five- or six-membered ring (which might be an aromatic or non-aromatic ring),
   R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and the remaining residues (i.e. those of the residues R³ to R⁶ which do not form a ring) are selected independently from each other from hydrogen and C₁₋₄alkyl;
   Lg¹ and Lg² are, independently from each other, a non-ionic ligand.
Embodiment {18} relates to the composition according to embodiment {17}, wherein
   L¹ is a C₁₋₃ alkylene group (e.g. -CH₂-; -CH₂-CH₂-; -CH₂-CH₂-CH₂-),
   Lg¹ is a pyridine, a pyrrole, an imidazole, a pyrazole or a pyrimidine, each of which is optionally substituted with one or more halogens,
   Lg² is an N-heterocyclic carbene.
Embodiment {19} relates to the composition according to embodiment {18}, wherein the N-heterocyclic carbene is of Formula (V) or Formula (VI): 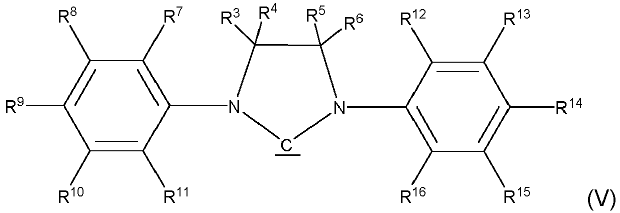 wherein in Formula (V)
   R³ to R¹⁶ are selected independently from each other from hydrogen and C₁₋₄alkyl; or at least two of the residues R³ to R¹⁶ which are adjacent to each other form together with the carbon atoms to which they are attached a 5- or 6-membered ring while the remaining residues (i.e. those of the residues R³ to R¹⁶ not forming a ring) are selected independently from each other from hydrogen and C₁₋₄alkyl; 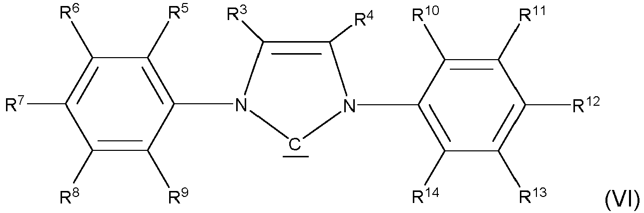 wherein in Formula (VI)
   R³ to R¹⁴ are selected independently from each other from hydrogen and C₁₋₄ alkyl; or at least two of the residues R³ to R¹⁴ which are adjacent to each other form together with the carbon atoms to which they are attached a 5- or 6-membered ring while the remaining residues (i.e. those of the residues R³ to R¹⁴ not forming a ring) are selected independently from each other from hydrogen and C₁₋₄alkyl.
Embodiment {20} relates to the composition according to one of the embodiments {17} to {19}, wherein the palladium complex is of Formula (IV-1): 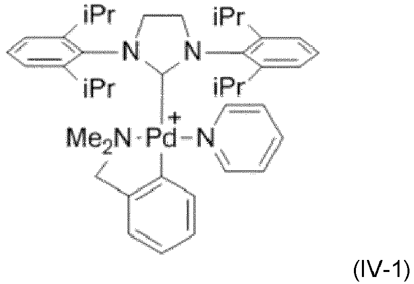
Embodiment {21} relates to the composition according to embodiment {16}, wherein the palladium complex is of Formula (VII) 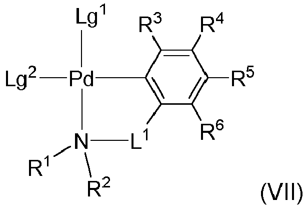 wherein
   L¹ is a bivalent linker,
   R¹ and R² are selected independently from each other from hydrogen and a C₁₋₄ alkyl group, or
   R¹ and R² together with the nitrogen atom to which they are attached form a five- or six-membered ring,
   R³, R⁴, R⁵, and R⁶ are selected independently from each other from hydrogen and C₁₋₄ alkyl, or at least two of the residues R³ to R⁶ which are adjacent to each other form together with the carbon atoms to which they are attached a five- or six-membered aromatic ring and the remaining residues (i.e. those of the residues R³ to R⁶ which do not form a ring) are selected independently from each other from hydrogen and C₁₋₄alkyl,
   Lg¹ is a non-ionic ligand,
   Lg² is an anionic ligand.
Embodiment {22} relates to the composition according to embodiment {21}, wherein
   L¹ is a phenylene (in particular a 1,2-phenylene) group or a C₁₋₃ alkylene group (e.g. -CH₂-; - CH₂-CH₂-; - CH₂-CH₂-CH₂-);
   the non-ionic ligand Lg¹ is a phosphine,
   the anionic ligand Lg² is an alkyl sulfonate (e.g. mesylate) or an aryl sulfonate (e.g. tosylate).
Embodiment {23} relates to the composition according to embodiment {22}, wherein the phosphine is of Formula (VIII):

   P(R¹)(R²)(R³) (VIII)

   wherein
   R¹ and R² are selected independently from each other from C₁₋₆ alkyl, cycloalkyl, and phenyl which is optionally substituted with one or more C₁₋₄ alkyl groups,
   R³ is biphenyl or binaphthyl, each of which is optionally substituted with one or more R⁴,
   R⁴, if present, is C₁₋₆ alkyl, -NH₂, -N(H)(C₁₋₆ alkyl), -N(C₁₋₆ alkyl)₂, -P(Phenyl)₂, or O-C₁₋₆alkyl.
Embodiment {24} relates to the composition according to embodiment {23}, wherein the palladium complex is selected from Formulas (VII-1) to (VII-7): 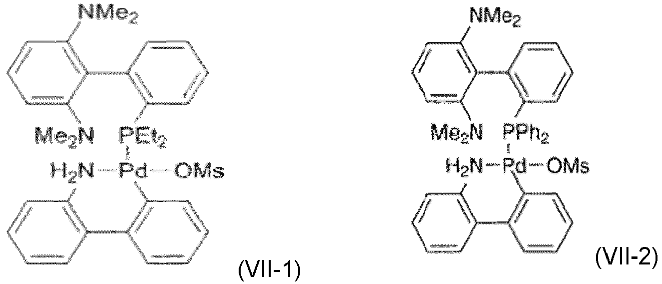 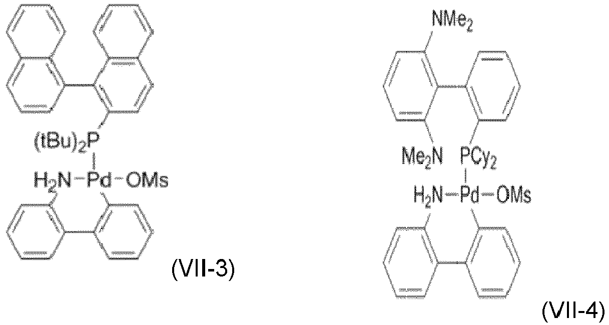 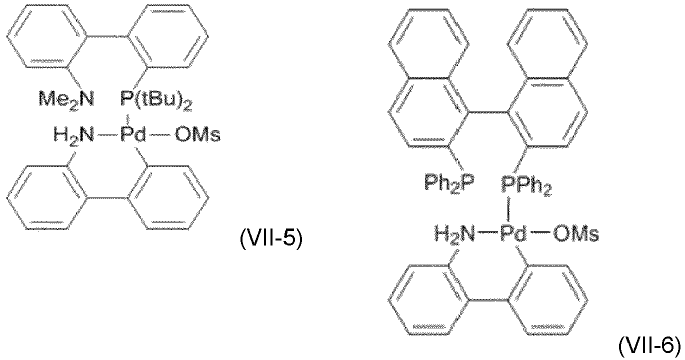 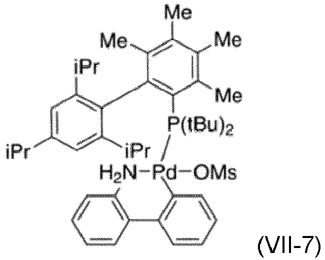
Embodiment {25} relates to the composition according to embodiment {22}, wherein the phosphine is a ferrocenyl phosphine.
Embodiment {26} relates to the composition according to embodiment {25}, wherein the palladium complex is of Formula (VII-8) or Formula (VII-9): 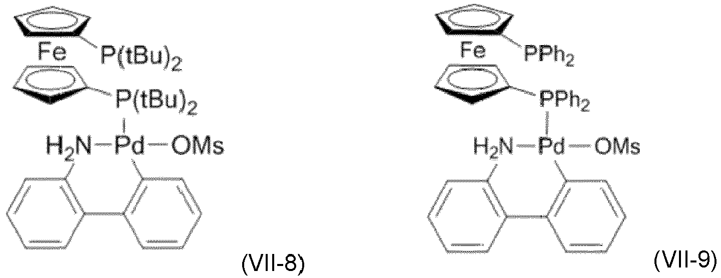
Embodiment {27} relates to the composition according to embodiment {22}, wherein the phosphine is a trialkyl phosphine (e.g. P(C₁₋₆ alkyl)₃).
Embodiment {28} relates to the composition according to embodiment {27}, wherein the palladium complex is of Formula (VII-10): 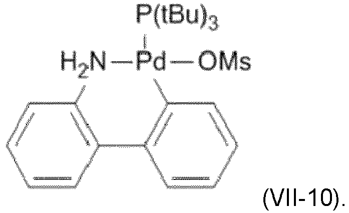
Embodiment {29} relates to the composition according to embodiment {22}, wherein the palladium complex is of Formula (VII-11), (VII-12), or (VII-13): 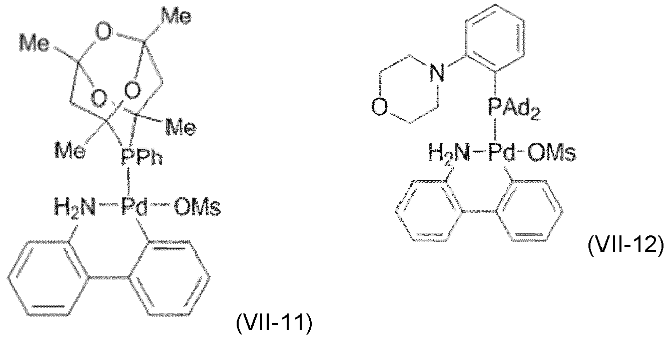 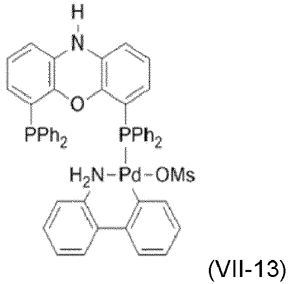
Embodiment {30} relates to the composition according to embodiment {16}, wherein the palladium complex is of Formula (IX):

   Pd(L)₃₋₄ (IX)

   wherein
   the ligands L, independently from each other, are a triaryl phosphine; preferably a triphenyl phosphine, wherein each aryl group is optionally substituted with one or more R¹,
   R¹, if present, is C₁₋₄ haloalkyl (e.g. -CF₃), C₁₋₄ alkyl (e.g. methyl), C₁₋₄ alkoxy (e.g. methoxy).
Embodiment {31} relates to the composition according to embodiment {30}, wherein the palladium complex is of Formula (IX-1) or (IX-2): 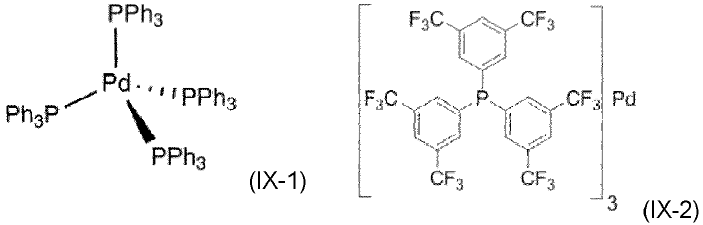
Embodiment {32} relates to the composition according to embodiment {16}, wherein the palladium complex is of Formula (X): 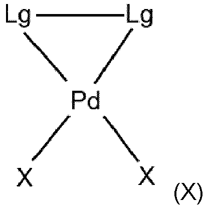 wherein
   Lg-Lg is a chelating ferrocenyl diphosphine ligand,
   X is a halide (e.g. CI).
Embodiment {33} relates to the composition according to embodiment {32}, wherein the palladium complex is of Formula (X-1) or (X-2): 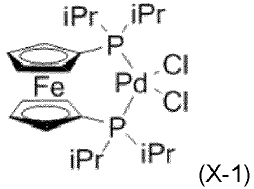 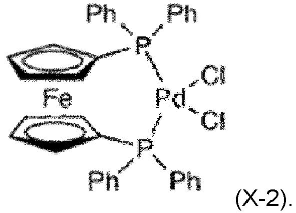
Embodiment {34} relates to the composition according to one of the embodiments {01} to {15}, further comprising, in addition to the palladium salt, a phosphine or a bipyridine (in particular a 2,2'-bipyridine).
Embodiment {35} relates to the composition according to embodiment {34}, wherein the phosphine is a tri-C₁₋₆alkyl phosphine, tri-C₅₋₇cycloalkyl phosphine, a triarylphosphine (in particular a triphenyl phosphine) wherein each of the aryl groups (which are preferably phenyl groups) is optionally substituted with one or more C₁₋₄ haloalkyl (e.g. -CF₃), C₁₋₄ alkyl (e.g. methyl) or C₁₋₄ alkoxy (e.g. methoxy).
Embodiment {36} relates to the composition according to embodiment {35}, wherein the phosphine is of Formula (XII-1), (XII-2), or (XII-3): 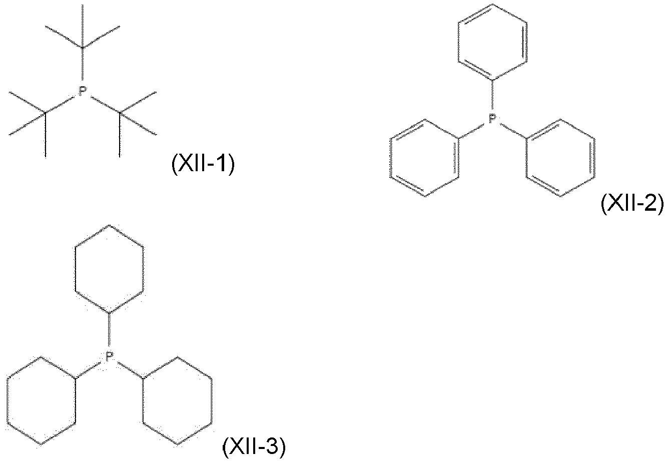
Embodiment {37} relates to the composition according to embodiment {34}, wherein the phosphine is of Formula (XIII):

   P(R¹)(R²)(R³) (XIII)

   wherein
   R¹ and R² are selected independently from each other from C₁₋₆ alkyl and C₅₋₇-cycloalkyl (e.g. cyclohexyl),
   R³ is biphenyl which is optionally substituted with one or more R⁴,
   R⁴, if present, is C₁₋₆ alkyl, C₁₋₆ alkoxy, phenyl, or pyridyl.
Embodiment {38} relates to the composition according to embodiment {37}, wherein the phosphine is of one of the Formulas (XIII-1) to (XIII-4): 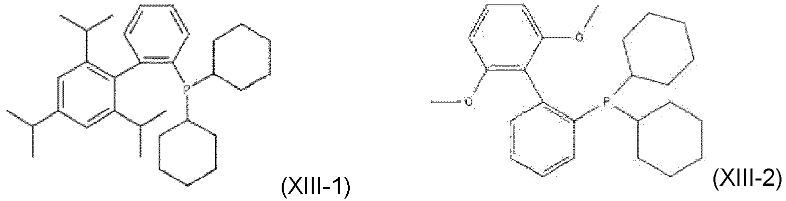 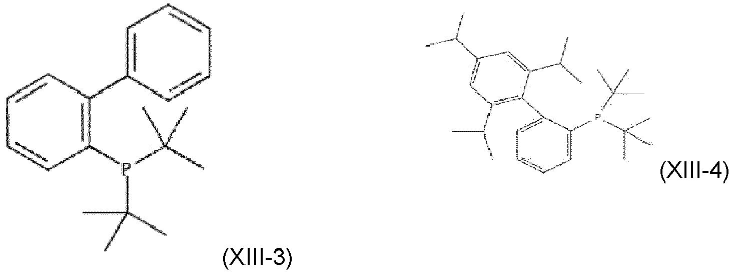
Embodiment {39} relates to the composition according to embodiment {34}, wherein the phosphine is a ferrocenyl diphosphine or a xanthenyl diphosphine.
Embodiment {40} relates to the composition according to embodiment {39}, wherein the phosphine is of one of the Formulas (XIV-1), (XIV-2) and (XV-1): 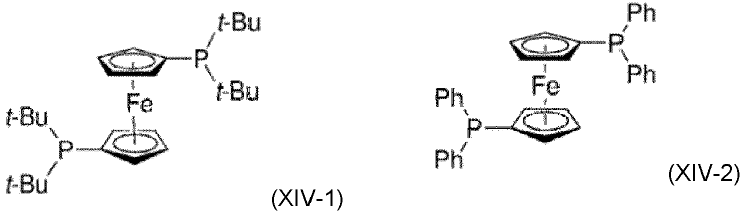 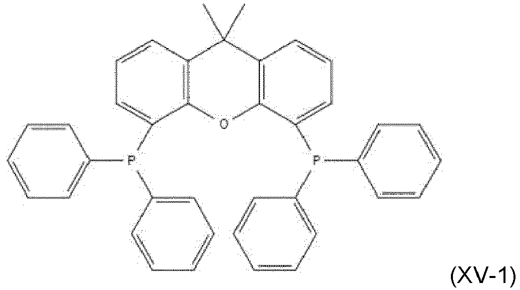
Embodiment {41} relates to the composition according to one of the embodiments {01} to {40}, wherein the composition comprises or is a mixture of the palladium compound and the polycyclic compound.
Embodiment {42} relates to the composition according to one of the preceding embodiments {01} to {41}, wherein the molar ratio between the palladium compound and the polycyclic compound is in the range of from 100/1 to 1/100, more preferably 10/1 to 1/10 or even more preferably 2/1 to 1/2
Embodiment {43} relates to the composition according to one of the embodiments {01} to {40}, wherein the composition is a kit which provides the palladium compound and the polycyclic compound in separate containers.
Embodiment {44} relates to a method of performing a Suzuki cross-coupling reaction, wherein a first reactant is reacted with a second reactant under irradiation of light in the presence of the composition according to one of the embodiments {01} to {42}, the first reactant comprising an aromatic or N-heteroaromatic ring to which a boronic acid or boronic acid ester group is attached, and the second reactant comprising an aromatic or N-heteroaromatic ring to which a halogen (CI, Br, I), a pseudohalogen (cyano, cyanate, isocyanate), or a sulfonate is attached.
Embodiment {45} relates to the method according to embodiment {44}, wherein the light has a wavelength in the range of from 254 nm to 800 nm.
Embodiment {46} relates to the method according to embodiment {44} or {45}, wherein the light is a monochromatic light; or the light covers a continuous wavelength spectrum (e.g. in the UV and/or visible spectrum).
Embodiment {47} relates to the method according to one of the embodiments {44} to {46}, wherein the light reaching the reaction medium has an irradiance of 10 to 2000 milliwatts per square centimeter.
Embodiment {48} relates to the method according to one of the embodiments {44} to {47}, wherein the first and second reactants are reacted in the presence of a base which is preferably selected from carbonate (e.g. an alkali metal carbonate), a hydroxide (e.g. an alkali metal hydroxide), a phosphate (e.g. an alkali metal phosphate), an alkoxide (e.g. an alkali metal alkoholate).
Embodiment {49} relates to the method according to one of the embodiments {44} to {48}, wherein the reaction medium in which the reaction is carried out comprises a solvent which is preferably selected from water, an aromatic solvent, an ether, a glycol ether, dimethyl formamide, acetonitrile, an alcohol, or a mixture of two or more of these solvents.
Embodiment {50} relates to the method according to one of the embodiments {44} to {49}, wherein the reactants are reacted at a temperature of from 20 °C to 70 °C.
Embodiment {51} relates to the use of the composition according to one of the embodiments {01} to {43} as a catalyst system for a light-assisted Suzuki carbon-carbon cross-coupling reaction.

## Claims

1. A composition, comprising
(i) a palladium compound which is selected from a palladium salt, a palladium complex or a mixture thereof,
(ii) a polycyclic compound of Formula (I), (II) or (III): 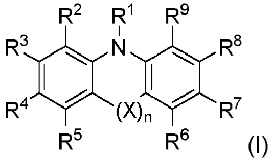 wherein
n is 0 or 1,
X is O, C(R¹⁰)(R¹¹), or N(R¹²),
R¹ to R¹² are selected independently from each other from hydrogen, an aryl group and a C₁₋₁₀alkyl group, the aryl group and/or the C₁₋₁₀alkyl group optionally being substituted with one or more substituents R¹³,
wherein the one or more substituents R¹³, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1) 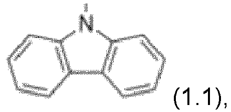 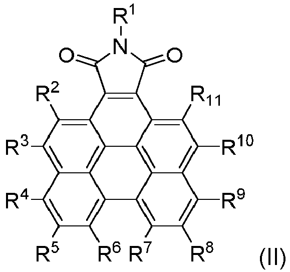 wherein
R¹ is selected from a C₁₋₁₆alkyl group and an aryl group, the C₁₋₁₆alkyl group and/or the aryl group optionally being substituted with one or more substituents R¹², and/or the C₁₋₁₆alkyl group optionally being interrupted by one or more oxygen atoms;
R² to R¹¹ are selected independently from each other from hydrogen, a C₁₋₁₆alkyl group, an aryl group, a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms; or
at least two of the residues R² to R¹¹ which are adjacent to each other form together with the carbon atoms to which they are attached a five- or six-membered aromatic ring while those of the residues R² to R¹¹ which do not form a ring are selected independently from each other from hydrogen, a C₁₋₁₆alkyl group, an aryl group and a heteroaryl group; the C₁₋₁₆alkyl group, the aryl group and/or the heteroaryl group optionally being substituted with one or more substituents R¹², and/or the C₁₋₁₆alkyl group optionally being interrupted by one or more oxygen atoms;
wherein the one or more substituents R¹², if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), 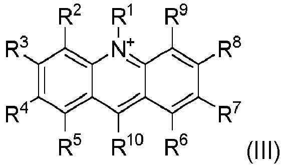 wherein
R¹ is selected from a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
R² to R⁹ are selected independently from each other from hydrogen, a C₁₋₁₆alkyl group and an aryl group, the C₁₋₁₆alkyl group and/or the aryl group optionally being substituted with one or more substituents R¹¹, and/or the C₁₋₁₆alkyl group optionally being interrupted by one or more oxygen atoms;
R¹⁰ is an aryl group which is optionally substituted with one or more substituents R¹², wherein the one or more substituents R¹¹, if present, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, - S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl),
wherein the one or more substituents R¹², if present, are selected independently from each other from a C₁₋₆alkyl group and an aryl group, wherein the aryl group is optionally substituted with one or more C₁₋₆alkyl groups.

2. The composition according to claim 1, wherein R¹ in Formula (I) is selected from a phenyl group, a naphthyl group and a biphenyl group, each of which is optionally substituted with one or more substituents R¹³, wherein R¹³ has the same meaning as in claim 1.

3. The composition according to claim 1 or 2, wherein in Formula (I)
n is 1,
X is O,
R¹ is a naphthyl group which is optionally substituted with one or more substituents R¹³, R², R³, R⁵, R⁶, R⁸ and R⁹ are selected independently from each other from hydrogen and a C₁₋₆alkyl group,
R⁴ and R⁷ are selected independently from each other from a phenyl group, a biphenyl group and a naphthyl group, each of which is optionally substituted with one or more substituents R¹³,
wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl),-C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

4. The composition according to claim 1 or 2, wherein in Formula (I)
n is 1,
X is C(R¹⁰)(R¹¹), wherein R¹⁰ and R¹¹ are selected independently from each other from hydrogen and a C₁₋₆alkyl group,
R¹ is selected from a naphthyl group, a phenyl group and a biphenyl group, each of which is optionally substituted with one or more substituents R¹³,
R², R³, R⁵, R⁶, R⁸ and R⁹ are selected independently from each other from hydrogen and a C₁₋₆alkyl group,
R⁴ and R⁷ are selected independently from each other from a phenyl group, a naphthyl group and a biphenyl group, each of which is optionally substituted with one or more substituents R¹³,
wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl) and -S(O)₂(phenyl).

5. The composition according to claim 1 or 2, wherein in Formula (I)
n is 1,
X is N(R¹²),
wherein
R¹ and R¹² are selected independently from each other from a phenyl group, a naphthyl group and a biphenyl group, each of which is optionally substituted with one or more substituents R¹³,
R² to R⁹ are selected independently from each other from hydrogen and a C₁₋₆alkyl group, or at least two of the residues R³, R⁴, R⁷, and R⁸ are a phenyl group which is optionally substituted with one or more substituents R¹³ and the residues R², R⁵, R⁶ and R⁹ are selected independently from each other from hydrogen and a C₁₋₆alkyl group, wherein, if present, the one or more substituents R¹³ are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), and -S(O)₂(phenyl).

6. The composition according to claim 1 or 2, wherein in Formula (I)
n is 0,
R¹ is selected from a naphthyl group, a phenyl group and a biphenyl group, each of which is optionally substituted with one or more R¹³,
R² to R⁹ are selected independently from each other from hydrogen, a C₁₋₆alkyl group, a phenyl group, biphenyl group and a naphthyl group, the phenyl group, the biphenyl group and/or the naphthyl group optionally being substituted with one or more substituents R¹³, wherein, if present, the one or more substituents R¹³, are selected independently from each other from C₁₋₆alkyl, C₁₋₆haloalkyl, -OH, -O-(C₁₋₆alkyl), -NO₂, -CN, a halogen atom, -NH₂, -NH(C₁₋₆alkyl), -N(C₁₋₆alkyl)₂, -C(=O)OH, -C(=O)O-(C₁₋₆alkyl), -C(=O)O-phenyl, -C(=O)(C₁₋₆alkyl), -C(=O)-phenyl, -S(O)₂NH₂, -S(O)₂NH(C₁₋₆alkyl), -S(O)₂N(C₁₋₆alkyl)(C₁₋₆alkyl), -S-(C₁₋₆alkyl), -S(O)(C₁₋₆alkyl), -S(O)₂(C₁₋₆alkyl), -S-(phenyl), -S(O)(phenyl), -S(O)₂(phenyl), and a N-carbazolyl of Formula (1.1)

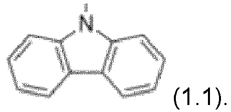

7. The composition according to claim 1 or 2, wherein in Formula (II)
R¹ is a C₁₋₁₂alkyl group which is optionally substituted with one or more substituents being selected from -OH, a halogen atom (e.g. F, CI), -NH₂, -NH(C₁₋₆alkyl) and -N(C₁₋₆alkyl)₂, R⁴, R⁷, and R⁹ are selected independently from each other from hydrogen, an aryl group, a heteroaryl group, C₁₋₆alkyl and -O-(C₁₋₆alkyl), the aryl group and/or the heteroaryl group optionally being substituted with one or more substituents which are selected independently from each other from C₁₋₆alkyl, C₁₋₆haloalkyl (e.g. C₁₋₆fluoroalkyl) and -O-(C₁₋₆alkyl),
R², R³, R⁵, R⁶, R⁸, R¹⁰, and R¹¹ are selected independently from each other from hydrogen and a C₁₋₆alkyl group.

8. The composition according to claim 1 or 2, wherein in Formula (III)
R¹ is a C₁₋₆alkyl group,
R¹⁰ is a phenyl group which is optionally substituted with one or more substituents being selected independently from each other from C₁₋₆alkyl, C₁₋₆haloalkyl and -O-(C₁₋₆alkyl), R² to R⁹ are selected independently from each other from hydrogen and a C₁₋₆alkyl group.

9. The composition according to one of the preceding claims, wherein the palladium salt is a palladium acetate, a palladium acetylacetonate or a palladium halide (such as palladium chloride (PdCl₂), palladium bromide (PdBr₂), or palladium iodide (PdI₂)), or any mixture thereof.

10. The composition according to one of the claims 1 to 8, wherein the palladium complex comprises one or more ligands which are selected from a phosphine, an N-heterocyclic carbene, an amine, or any combination thereof.

11. The composition according to claim 10, wherein the palladium complex is of Formula (IV) 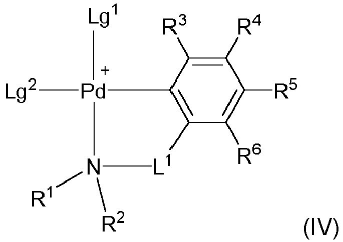 wherein
L¹ is a bivalent linker,
R¹ and R² are selected independently from each other from hydrogen and a C₁₋₄alkyl group, or R¹ and R² together with the nitrogen atom to which they are attached form a five- or six-membered ring,
R³, R⁴, R⁵, and R⁶ are selected independently from each other from hydrogen and C₁₋₄alkyl; or at least two of the residues R³ to R⁶ which are adjacent to each other form together with the carbon atoms to which they are attached a five- or six-membered aromatic ring and those of the residues R³ to R⁶ which do not form a ring are selected independently from each other from hydrogen and C₁₋₄alkyl;
Lg¹ and Lg² are, independently from each other, a non-ionic ligand.

12. The composition according to claim 11, wherein
L¹ is a C₁₋₃ alkylene group,
Lg¹ is a pyridine, a pyrrol, an imidazole, a pyrazole or a pyrimidine, each of which is optionally substituted with one or more halogens,
Lg² is an N-heterocyclic carbene.

13. The composition according to claim 10, wherein the palladium complex is of Formula (VII) 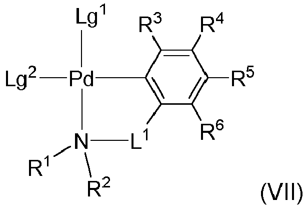 wherein
L¹ is a bivalent linker,
R¹ and R² are selected independently from each other from hydrogen and a C₁₋₄ alkyl group, or R¹ and R² together with the nitrogen atom to which they are attached form a five- or six-membered ring,
R³, R⁴, R⁵, and R⁶ are selected independently from each other from hydrogen and C₁₋₄alkyl, or at least two of the residues R³ to R⁶ which are adjacent to each other form together with the carbon atoms to which they are attached a five- or six-membered aromatic ring and those of the residues R³ to R⁶ which do not form a ring are selected independently from each other from hydrogen and C₁₋₄alkyl,
Lg¹ is a non-ionic ligand,
Lg² is an anionic ligand.

14. The composition according to claim 13, wherein
L¹ is a phenylene group or a C₁₋₃ alkylene group;
the non-ionic ligand Lg¹ is a phosphine,
the anionic ligand Lg² is an alkyl sulfonate or an aryl sulfonate.

15. The composition according to claim 10, wherein the palladium complex is of Formula (IX) or (X):
Pd(L)₃₋₄ (IX)
wherein in Formula (IX)
the ligands L, independently from each other, are a triaryl phosphine, wherein each aryl group is optionally substituted with one or more R¹,
wherein, if present, the one or more R¹ are selected independently from each other from C₁₋₄ haloalkyl, C₁₋₄alkyl and C₁₋₄ alkoxy; 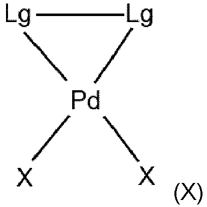 wherein in Formula (X)
Lg-Lg is a chelating ferrocenyl diphosphine ligand,
X is a halide.

16. The composition according to one of the claims 1 to 9, further comprising, in addition to the palladium salt, a phosphine or a bipyridine.

17. The composition according to one of the preceding claims, wherein the composition comprises or is a mixture of the palladium compound and the polycyclic compound, the molar ratio of the palladium compound and the polycyclic compound preferably being in the range of 100/1 to 1/100.

18. The composition according to one of the claims 1 to 16, wherein the composition is a kit which provides the palladium compound and the polycyclic compound in separate containers.

19. A method of performing a Suzuki cross-coupling reaction, wherein a first reactant is reacted with a second reactant under irradiation of light in the presence of the composition according to one of the claims 1 to 17, the first reactant comprising an aromatic or N-heteroaromatic ring to which a boronic acid or boronic acid ester group is attached, and the second reactant comprising an aromatic or N-heteroaromatic ring to which a halogen, a pseudohalogen or a sulfonate is attached.
